# EUROPEAN PATENT APPLICATION

(11) **EP 4 835 098 A1**
(43) Date of publication of application: **04.11.2026**
(21) Application number: 23962957.9
(22) Date of filing: 29.12.2023
(51) Int. Cl.: C12Q 1/6874, C12M 1/34, C12Q 1/6844, C12Q 1/6841, C12Q 1/6876, C12Q 1/686, C12Q 1/6806, C12N 15/11, C40B 40/06, C40B 20/02

(54) **ARRAY AND METHOD FOR DETECTING SPATIAL INFORMATION OF NUCLEIC ACIDS**

(71) Applicant: STOmics Tech Co., Ltd., Shenzhen, Guangdong 518085 (CN)
(72) Inventor: LIAO, Sha, Shenzhen, Guangdong 518083 (CN); CHEN, Ao, Shenzhen, Guangdong 518083 (CN); XU, Xun, Shenzhen, Guangdong 518083 (CN); ZHANG, Xue, Shenzhen, Guangdong 518083 (CN); JIANG, Xia, Shenzhen, Guangdong 518083 (CN); ZHANG, Wenwei, Shenzhen, Guangdong 518083 (CN); HE, Ying, Shenzhen, Guangdong 518083 (CN)
(74) Representative: Inspicos P/S
(86) International application number: PCT/CN2023/143677
(87) International publication number: WO 2025/138284

(57) **Abstract**

The present application provides a nucleic acid array for detecting the spatial information of nucleic acids in a sample, a method for detecting spatial information of nucleic acids in a sample on the basis of the array, and a method for producing the nucleic acid array.

## Description

### Technical Field

The present application relates to the field of spatial detection of biomolecules. Specifically, the present application provides a nucleic acid array for detecting the spatial information of nucleic acid in a sample, a method for detecting the spatial information of nucleic acid in a sample based on the array, and a method for generating the nucleic acid array.

### Background Art

Spatial omics technology is a technique that enables the acquisition of omics information such as transcriptome, genome, epigenome, and proteome from cells in situ within tissues. Spatial omics technology was named the Method of the Year by Nature Methods in 2020 and was listed as one of Nature's seven most noteworthy technologies in 2022. Based on different principles, spatial omics technology can be classified into four categories: those based on microdissection, in situ hybridization, in situ sequencing, and array-based in situ capture combined with high-throughput sequencing. Among these, the approach combining array-based in situ capture and high-throughput sequencing is dominant due to its ease of operation and high throughput, with the capture array being a key component of this technology.

Array-based spatial omics technology involves the in situ capture of nucleic acids and other molecules from tissue sections using probes carrying spatial information on an array. High-throughput sequencing is then applied to obtain spatial information and intracellular nucleic acid molecular information from the array, followed by algorithmic analysis to reconstruct the molecular information within the tissue. Since 2016, when Joakim Lundeberg from the Royal Institute of Technology in Sweden first published spatial transcriptomics technology in Science, the field has developed rapidly due to its advantages such as high-throughput whole-transcriptome analysis. Other similar spatial transcriptomics technologies include HDST (High-definition spatial transcriptomics) based on silica magnetic beads, Slide-seq (sequencing by oligonucleotide ligation and detection) developed using barcoded microbeads, DBiT-seq (Deterministic Barcoding in Tissue) based on microfluidics, Seq-Scope based on the Illumina sequencing platform, and Stereo-seq (spatial enhanced resolution omics sequencing) based on the DNB sequencing platform. Among these, Joakim's spatial transcriptomics technology was acquired and commercialized by 10x Genomics at the end of 2018, and the Visium product was released in 2019. To date, 10x Genomics has developed a spatial omics product line encompassing instruments, analytical software, reagents and consumables, in addition to its single-cell product line. Meanwhile, Biomarker Technologies also launched a spatial transcriptomics product based on this technology in 2022.

The core of existing spatial omics technologies employing array-based in situ capture lies in the resolution and capture efficiency of the capture array. The resolution of the array is determined by the spatial information density on the array, which affects whether fine-resolution analysis at the single-cell or subcellular level can be achieved for cells in tissue sections. Capture efficiency, on the other hand, is determined by the capture information on the array, which affects whether all molecular information within a cell can be comprehensively captured without bias. In existing technologies that use sequencing arrays as capture arrays, the capture functional region and the spatial information region reside on the same molecule of the nucleic acid array. However, limited by the resolution of the sequencing array, nucleic acid molecules comprising spatial information regions in the nucleic acid array must be spaced at a certain distance apart to be recognized by the sequencer. If the distance is too small, the individual nucleic acid molecules cannot be accurately identified. When the capture functional region and the spatial information region are on the same nucleic acid molecule, resolution is constrained, and consequently, capture efficiency is also limited.

Furthermore, since existing capture arrays rely on poly-T probes for mRNA capture, they can only capture transcripts from the 3' end to simultaneously obtain both the expression pattern and spatial information of transcripts, thereby losing information from the 5' end. For the immune repertoire, however, key molecular information is concentrated at the 5' end, revealing a significant limitation of current technologies in capturing 5' end transcripts.

### Contents of the Disclosure

To simultaneously achieve high resolution and high capture efficiency in spatial omics technologies, the present application decouples the capture region and spatial information region present on the capture array probes in existing technologies, designing them instead as two separate molecules: a positioning probe and a capture probe, in which the positioning probe provides spatial positioning information for spatial omics, while the capture probe captures intracellular molecules, thereby overcoming the limitation that the number of capture probes is constrained by spatial density. This enables improved capture efficiency of molecules while maintaining high resolution.

Furthermore, the nucleic acid array (e.g., capture array) provided in the present application also allows for the acquisition of 5' transcript information.

### Nucleic acid array

Therefore, in one aspect, the present application provides a nucleic acid array for detecting spatial information of nucleic acid in a sample, which comprises a solid support to which one or more types of capture probes and at least two types of positioning probes are attached;
the capture probes and the positioning probes are each independently attached to the solid support;
each type of the positioning probe occupies a different position on the solid support;
the positioning probe comprises a first universal sequence and a positioning sequence, wherein the positioning sequence has a nucleotide sequence corresponding to the position of the type of positioning probe on the solid support;
the capture probe comprises a capture sequence, wherein the capture sequence is capable of annealing to the nucleic acid molecule to be captured and initiating an extension reaction.

In some embodiments, each type of the positioning probe consists of one or more positioning probe molecules.

In some embodiments, the positioning sequences comprised in the positioning probe molecules of the same type of positioning probes are identical to each other, while the positioning sequences comprised in the positioning probe molecules of different types of positioning probes are different from each other.

It will be readily understood by those skilled in the art that one or more positioning probe molecules of the same type of positioning probes share the same positioning sequence; however, it is not required that every positioning probe molecule of each type of positioning probes has the same complete sequence.

In some embodiments, the positioning probe molecule and the capture probe of the nucleic acid array are separate.

In some embodiments, the first universal sequence is located 3' of the positioning sequence.

In some embodiments, the positioning probe further comprises a second universal sequence.

In some embodiments, the second universal sequence is located 5' of the positioning sequence.

In some embodiments, the second universal sequences comprised in different types of positioning probes attached to the solid support are the same or different. In some embodiments, the second universal sequences comprised in different types of positioning probes attached to the solid support are the same. In some embodiments, the second universal sequences comprised in the same type of positioning probes attached to the solid support are the same or different.

In some embodiments, the first universal sequences comprised in the same type of positioning probes attached to the solid support are the same or different.

In some embodiments, the positioning probe does not comprise or further comprises a molecular identifier (MID).

In some embodiments, the positioning probe further comprises an MID sequence.

In some embodiments, the MID sequence is located 5' of the first universal sequence, and/or, the MID sequence is located 3' of the second universal sequence.

In some embodiments, the MID sequences comprised in the same type of positioning probes are different from each other.

In some embodiments, the capture sequence is located at the 3' end of the capture probe.

In some embodiments, the capture sequence has a free hydroxyl (-OH) at the 3' end.

In some embodiments, the capture probe further comprises a consensus sequence.

In some embodiments, the consensus sequences comprised in different capture probes attached to the solid support are the same or different. In some embodiments, the consensus sequences comprised in different capture probes attached to the solid support are the same.

In some embodiments, the consensus sequence is located 5' of the capture sequence.

In some embodiments, the capture probe comprises a single-stranded region that comprises the capture sequence.

In some embodiments, the capture probe is present in a single-stranded form or duplex form that comprises the single-stranded region.

In some embodiments, the capture probe is present in a single-stranded form, and the capture sequence is located at the 3' end of the capture probe; or,
the capture probe is present in a duplex form that comprises a single-stranded region, comprising a first strand directly attached to the solid support, and a second strand hybridized to the first strand; and, the 3' end of the second strand comprises a single-stranded region, and the capture sequence is located at the 3' end of the single-stranded region.

In some embodiments, the second strand comprises the capture sequence and the consensus sequence.

In some embodiments, the nucleic acid array has one or more features selected from the following:
(1) the positioning sequence is a nucleotide sequence consisting of 5 to 50 (e.g., 5 to 25, 5 to 35, 5 to 45, 10 to 25, 10 to 35, 10 to 45, 15 to 25, 15 to 35, 15 to 45, 20 to 25, 20 to 35, or 20 to 45) random nucleotides; in some embodiments, each random nucleotide is independently any one of deoxyribonucleotides A, C, G, and T;
(2) the solid support is selected from the group consisting of latex bead, dextran bead, polystyrene surface, polypropylene surface, polyacrylamide gel, gold surface, glass surface, chip, sensor, electrode, and silicon wafer; in some embodiments, the solid support is a chip; in some embodiments, the solid support can be used in a sequencing platform; in some embodiments, the solid support is a sequencing chip (e.g., a high-throughput sequencing chip), such as a sequencing chip (e.g., a high-throughput sequencing chip) for use in an Illumina, MGI, or Thermo Fisher sequencing platform;
(3) positioning probes of the same type (i.e., positioning probes comprising the same positioning sequence) occupy the same region on the surface of the solid support, positioning probes of different types occupy different regions on the surface of the solid support, and the center-to-center distance between any two adjacent regions is less than 1 µm (e.g., less than 900 nm, less than 800 nm, less than 700 nm, less than 600 nm, less than 500 nm, less than 400 nm, less than 300 nm, less than 200 nm);
(4) the first universal sequence is capable of annealing to (i) the nucleic acid molecule captured by the capture sequence, or (ii) a nucleic acid molecule derived from (i);
(5) the first universal sequences comprised in different types of positioning probes attached to the solid support are the same or different;
(6) the solid support is capable of releasing the positioning probe and/or capture probe spontaneously or upon exposure to one or more stimuli (e.g., temperature change, pH change, exposure to a specific chemical or phase, exposure to light, a reducing agent, etc.).

In some embodiments, the positioning probe and/or the capture probe are covalently or non-covalently attached to the solid support.

In some embodiments, the positioning probe and/or the capture probe are covalently attached to the solid support.

In some embodiments, the positioning probe and the capture probe are each independently attached to the solid support via the same or different click chemistry reactions.

In some embodiments, the molecule or group pair capable of undergoing the click chemistry reaction is selected from the group consisting of: alkynyl/azido, azido/cyano, amine/ene, thiol/ene, thiol/alkynyl, aldehyde/1,3-diol, ketone/1,3-diol. In some embodiments, the molecule or group pair capable of undergoing the click chemistry reaction is azido/alkynyl.

In some embodiments, the solid support is surface-modified with a molecule or group X, and the positioning probe is modified with a molecule or group Y, wherein the molecule or group X is capable of undergoing a click chemistry reaction with the molecule or group Y, and the positioning probe is attached to the solid support via the click chemistry reaction between the molecule or group X and the molecule or group Y; and/or, the solid support is surface-modified with the molecule or group X', and the capture probe is modified with a molecule or group Y', wherein the molecule or group X' is capable of undergoing a click chemistry reaction with the molecule or group Y', and the capture probe is attached to the solid support via the click chemistry reaction between the molecule or group X' and the molecule or group Y'.

In some embodiments, the molecule or group pairs X-Y and X'-Y' are each independently selected from the group consisting of: alkynyl/azido, azido/cyano, amine/ene, thiol/ene, thiol/alkynyl, aldehyde/1,3-diol, ketone/1,3-diol, azido/alkynyl, cyano/azido, ene/amine, ene/thiol, alkynyl/thiol, 1,3-diol/aldehyde, 1,3-diol/ketone.

In some embodiments, the molecule or group X and/or X' is azido, and the molecule or group Y and/or Y' is

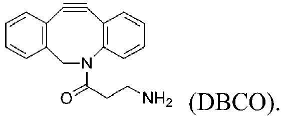

In some embodiments, the solid support is surface-modified with azido, the positioning probe and/or the capture probe are modified with 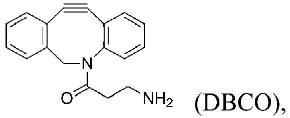 and the positioning probe and/or the capture probe are attached to the solid support via a click chemistry reaction between the azido and the DBCO.

It will be readily appreciated by those skilled in the art that any part of the positioning probe can be used for attachment to the solid support, as long as such attachment does not affect the ability of the positioning probe to perform its functions (e.g., annealing to a target sequence and/or initiating an extension reaction from the 3' end). In some embodiments, the positioning probe is capable of annealing to a target sequence and the 3' end of the positioning probe is capable of initiating an extension reaction; in such cases, any part of the positioning probe can be used for attachment to the solid support, provided that the attachment does not affect the annealing of the positioning probe to the target sequence and/or the initiation of the extension reaction from the 3' end. In some embodiments, the positioning probe is capable of annealing to a target sequence and the 3' end of the positioning probe is blocked; in such cases, any part of the positioning probe can be used for attachment to the solid support, as long as the attachment does not affect the annealing of the positioning probe to the target sequence. In some embodiments, the positioning sequence of the positioning probe is not directly attached to the solid support. In some embodiments, the first universal sequence of the positioning probe is not directly attached to the solid support. In some embodiments, neither the first universal sequence nor the positioning sequence of the positioning probe is directly attached to the solid support. In some embodiments, the positioning probe is attached to the solid support via a second universal sequence. In some embodiments, the positioning probe is attached to the solid support via its 5' end (e.g., the 5' terminus).

It will be readily appreciated by those skilled in the art that any part of the capture probe can be used for attachment to the solid support, as long as such attachment does not affect the ability of the capture probe to perform its functions (e.g., annealing to a target sequence and initiating an extension reaction from the 3' end). In some embodiments, the capture probe is present in single-stranded form, in which case any part of the capture probe can be used for attachment to the solid support, provided that such attachment does not affect the functions of annealing to the target sequence and initiating the extension reaction from the 3' end. In some embodiments, the capture sequence of the capture probe is not directly attached to the solid support. In some embodiments, the capture probe is attached to the solid support via its 5' end (e.g., the 5' terminus).

In some embodiments, the capture probe is present in a duplex form comprising a single-stranded region, the duplex comprising a first strand directly attached to the solid support and a second strand hybridized to the first strand, in which case any part of the first strand can be used for attachment to the solid support, as long as such attachment does not affect the ability of the first strand to perform its functions (e.g., the function of annealing to the second strand). In some embodiments, the first strand is attached to the solid support via a portion that is not annealed to the second strand. In some embodiments, the first strand is attached to the solid support via its terminus (e.g., the 5' terminus or the 3' terminus). In some embodiments, the second strand is not directly attached to the solid support.

In some embodiments, the distribution of the capture probes and the positioning probes on the solid support is configured such that a nucleic acid molecule linked to the capture probe (e.g., a nucleic acid molecule annealed to the capture probe, or a nucleic acid molecule obtained by extension of the capture probe via a nucleic acid polymerization reaction) is capable of contacting a positioning probe in proximity thereto.

In some embodiments, at least 100 of the capture probes are distributed in proximity to each type of the positioning probes. For example, there are at least 100, at least 200, at least 300, at least 400, at least 500, at least 600, at least 700, at least 800, at least 900, at least 1000, at least 1500, at least 2000, at least 2500, at least 3000, at least 3500, at least 4000, at least 4500, at least 5000, at least 5500, at least 6000, at least 6500, at least 7000, at least 7500, at least 8000, at least 8500, at least 9000, at least 9500, or at least 10000 of the described capture probes.

In some embodiments, there are 500 to 10000 (e.g., 1000 to 1500, 1000 to 2000, 1000 to 3000, 1000 to 4000, 1000 to 5000, 1000 to 8000, or 1000 to 10000) of the capture probes that are distributed in proximity to each type of the positioning probes.

In some embodiments, the expression "in proximity to each type of the positioning probes" refers to a region on the surface of the solid support adjacent to the type of positioning probes, wherein nucleic acid molecules linked to the capture probes distributed in the region (e.g., nucleic acid molecules annealed to the capture probes, or nucleic acid molecules obtained by extension of the capture probes via a nucleic acid polymerization reaction) are capable of contacting the type of positioning probes.

In some embodiments, at least 100 of the capture probes are distributed around the center of the location occupied by each type of the positioning probes on the solid support (e.g., the center of the area occupied by each type of the positioning probes on the surface of the solid support) within a radius of 200 nm to 300 nm. For example, there are at least 100, at least 200, at least 300, at least 400, at least 500, at least 600, at least 700, at least 800, at least 900, at least 1000, at least 1500, at least 2000, at least 2500, at least 3000, at least 3500, at least 4000, at least 4500, at least 5000, at least 5500, at least 6000, at least 6500, at least 7000, at least 7500, at least 8000, at least 8500, at least 9000, at least 9500, or at least 10000 of the described capture probes.

In some embodiments, 500 to 10000 (e.g., 1000 to 1500, 1000 to 2000, 1000 to 3000, 1000 to 4000, 1000 to 5000, 1000 to 8000, 1000 to 10000) of the capture probes are distributed within a radius of 200 to 300 nm around the center of the location occupied by each type of the positioning probes on the solid support (e.g., the center of the area occupied by each type of the positioning probes on the surface of the solid support).

As used herein, the expression "the area occupied by each type of the positioning probes on the surface of the solid support" or similar expressions have the same meaning as "the location occupied by each type of the positioning probes on the solid support" and are used interchangeably.

In some embodiments, the nucleic acid array comprises one or more types of the capture probes.

In some embodiments, different types of the capture probes comprise different capture sequences.

In some embodiments, the nucleic acid molecule to be captured is an RNA (e.g., an mRNA), and the capture sequence of the capture probe comprises a poly(dT) sequence or a random oligonucleotide sequence;
the nucleic acid molecule to be captured is a target nucleic acid (e.g., a target DNA and/or RNA) or a nucleic acid molecule derived from the target nucleic acid, the target nucleic acid or the nucleic acid molecule derived from the target nucleic acid has a target nucleotide sequence comprised in the target nucleic acid, and the capture sequence of the capture probe comprises a sequence capable of specifically annealing to the target nucleotide sequence; or,
the nucleic acid molecule to be captured is an RNA (e.g., an mRNA) and a target nucleic acid (e.g., a target DNA and/or RNA), the nucleic acid array comprises a first capture probe for capturing RNA (e.g., mRNA) and a second capture probe for capturing the target nucleic acid or a nucleic acid molecule derived from the target nucleic acid, and the target nucleic acid or the nucleic acid molecule derived from the target nucleic acid has a target nucleotide sequence comprised in the target nucleic acid; wherein the capture sequence of the first capture probe comprises a poly(dT) sequence or a random oligonucleotide sequence, and the capture sequence of the second capture probe comprises a sequence capable of specifically annealing to the target nucleotide sequence.

It will be readily understood by those skilled in the art that, unless otherwise specified herein or clearly contradicted in the context, the definitions/descriptions of the capture probe as provided above also apply to the first capture probe and the second capture probe.

In some embodiments, the spatial information of the nucleic acid comprises the location, distribution, and/or abundance of the nucleic acid.

In some embodiments, the first universal sequence is a nucleotide sequence consisting of 5 to 80 (e.g., 5 to 20, 5 to 30, 5 to 40, 5 to 50, 5 to 70, 10 to 30, 10 to 50, 10 to 70, 20 to 30, 20 to 50, 20 to 70, 25 to 40, 25 to 50, 25 to 70) nucleotides.

In some embodiments, the second universal sequence is a nucleotide sequence consisting of 5 to 80 (e.g., 5 to 20, 5 to 30, 5 to 40, 5 to 50, 5 to 70, 10 to 30, 10 to 50, 10 to 70, 20 to 30, 20 to 50, 20 to 70, 25 to 40, 25 to 50, 25 to 70) nucleotides.

In some embodiments, the consensus sequence is a nucleotide sequence consisting of 5 to 80 (e.g., 5 to 20, 5 to 30, 5 to 40, 5 to 50, 5 to 70, 10 to 30, 10 to 50, 10 to 70, 20 to 30, 20 to 50, 20 to 70, 25 to 40, 25 to 50, 25 to 70) nucleotides.

In some embodiments, the capture sequence is a nucleotide sequence consisting of 5 to 50 (e.g., 5 to 25, 5 to 35, 5 to 45, 10 to 25, 10 to 35, 10 to 45, 15 to 25, 15 to 35, 15 to 45, 20 to 25, 20 to 35, or 20 to 45) nucleotides.

In some embodiments, the random oligonucleotide sequence is a nucleotide sequence consisting of 5 to 50 (e.g., 5 to 25, 5 to 35, 5 to 45, 10 to 25, 10 to 35, 10 to 45, 15 to 25, 15 to 35, 15 to 45, 20 to 25, 20 to 35, or 20 to 45) random nucleotides. In some embodiments, each random nucleotide is independently any one of deoxyribonucleotides A, C, G, and T.

In some embodiments, the poly(dT) sequence consists of 5 to 50 (e.g., 5 to 25, 5 to 35, 5 to 45, 10 to 25, 10 to 35, 10 to 45, 15 to 25, 15 to 35, 15 to 45, 20 to 25, 20 to 35, or 20 to 45) thymine deoxyribonucleotide residues.

In some embodiments, the first universal sequence, the positioning sequence, the second universal sequence, the consensus sequence, and the capture sequence each independently comprises or does not comprise a non-natural nucleotide residue (e.g., a modified nucleotide residue).

### Preparation method of nucleic acid array

In another aspect, the present application provides a method for preparing a nucleic acid array as described above, which comprises the following:
(A) attaching and/or synthesizing a positioning probe on a solid support, wherein the positioning probe is as defined above; and,
(B) attaching and/or synthesizing a capture probe on a solid support, wherein the capture probe is as defined above;
wherein, the (A) and the (B) can be performed in any order or simultaneously (e.g., simultaneously in the same reaction system).

In some embodiments, the (A) is performed before the (B), and the solid support in the (B) is a solid support already attached with the positioning probe.

In some embodiments, the (B) is performed before the (A), and the solid support in the (A) is a solid support already attached with the capture probe.

In some embodiments, the (A) and the (B) are performed simultaneously, and the solid support in both the (A) and the (B) is the same solid support.

In some embodiments, the (A) comprises:
(1) providing: (a) the positioning probe in free form, wherein the positioning probe is modified with a molecule or group A; and (b) the solid support, wherein the solid support is surface-modified with a molecule or group B, wherein the molecule or group A is capable of forming a linkage (e.g., covalent or non-covalent linkage) with the molecule or group B; and,
(2) contacting the positioning probe with the solid support under a condition suitable for forming a linkage between the molecule or group A and the molecule or group B, thereby obtaining the solid support with the positioning probe attached.

In some embodiments, the (A) further comprises step (3): performing bridging amplification of the positioning probe on the solid support with the positioning probe attached, thereby obtaining multiple copy clusters of each type of positioning probe.

It will be appreciated by those skilled in the art that any part of the positioning probe can be modified with the molecule or group A, as long as it does not affect the ability of the positioning probe to perform its function (e.g., annealing to a target sequence and/or initiating an extension reaction from the 3' end). In some embodiments, the positioning probe is capable of annealing to a target sequence and the 3' end of the positioning probe is capable of initiating an extension reaction; in such cases, any part of the positioning probe can be modified with the molecule or group A, as long as it does not affect the annealing of the positioning probe to the target sequence and/or the initiation of the extension reaction from the 3' end. In some embodiments, the positioning probe is capable of annealing to a target sequence and the 3' end of the positioning probe is blocked; in such cases, any part of the positioning probe can be modified with the molecule or group A, as long as it does not affect the annealing of the positioning probe to the target sequence. In some embodiments, the positioning sequence of the positioning probe is not modified with the molecule or group A. In some embodiments, the first universal sequence of the positioning probe is not modified with the molecule or group A. In some embodiments, neither the first universal sequence nor the positioning sequence of the positioning probe is modified with the molecule or group A. In some embodiments, the second universal sequence of the positioning probe is modified with the molecule or group A. In some embodiments, the 5' end (e.g., the 5' terminus) of the positioning probe is modified with the molecule or group A.

In some embodiments, the molecule or group A is capable of undergoing a click chemistry reaction with the molecule or group B.

In some embodiments, the molecule or group pair A/B is selected from the group consisting of: alkynyl/azido, azido/cyano, amine/ene, thiol/ene, thiol/alkynyl, aldehyde/1,3-diol, ketone/1,3-diol, azido/alkynyl, cyano/azido, ene/amine, ene/thiol, alkynyl/thiol, 1,3/diol-aldehyde, 1,3/diol-ketone.

In some embodiments, the molecule or group pair A/B is azido/alkynyl or alkynyl/azido.

In some embodiments, the molecule or group A is DBCO, and the molecule or group B is azido.

In some embodiments, the positioning probe is modified with 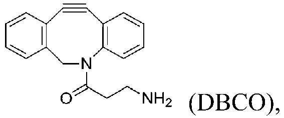 the solid support is surface-modified with azido, and the positioning probe is attached to the solid support via a click chemistry reaction between DBCO and azido.

In some embodiments, the (A) comprises:
(1) providing at least two types of carriers, each type of carrier comprising at least one copy of a carrier sequence, wherein the carrier sequence comprises: a complementary sequence to a positioning sequence and a complementary sequence to a first universal sequence; the first universal sequence and the positioning sequence are as defined above; wherein the complementary sequences to the positioning sequences of each type of the carrier sequences are different from each other;
(2) placing at least two types of the carriers onto the surface of the solid support;
(3) providing a immobilization primer, and using the carrier sequence as a template to carry out a nucleic acid polymerization reaction to generate an extension product, wherein the extension product is the positioning probe; wherein the immobilization primer is capable of annealing to the carrier sequence and initiating an extension reaction; and
(4) attaching the immobilization primer to the surface of the solid support;
wherein steps (3) and (4) are performed in any order (for example, step (3) is performed before or after step (4), or step (3) and step (4) are performed simultaneously).

In some embodiments, in step (2), the carriers are attached to the surface of the solid support by forming a linkage (for example, non-covalent or covalent linkage), thereby being placed on the surface of the solid support.

In some embodiments, the carrier sequence comprises, in the direction from 5' to 3', the complementary sequence to the universal sequence and the complementary sequence to the positioning sequence.

In some embodiments, the extension product comprises, in the direction from 5' to 3', the positioning sequence and the first universal sequence.

In some embodiments, each type of carrier is a DNB formed by a concatemer of multiple copies of carrier sequence.

In some embodiments, the method optionally comprises step (5): digesting the carrier sequence, and/or separating (e.g., by melting) the extension product in step (3) from the carrier sequence annealed thereto.

In some embodiments, the carrier sequence further comprises a cleavage site. In some embodiments, the cleavage site can be cleaved by a method selected from the group consisting of nicking enzyme digestion, USER digestion, photocleavage, chemical cleavage, or CRISPR cleavage. In some embodiments, in step (5), the carrier sequence is separated from the extension product of step (3) annealed thereto by cleaving the cleavage site comprised in the carrier sequence.

In some embodiments, in step (1), the carrier is provided by the following steps:
(i) providing a template of the carrier sequence, wherein the template comprises a complementary sequence to the carrier sequence;
(ii) using the template as a template, performing a nucleic acid amplification reaction to obtain an amplification product of the template, wherein the amplification product comprises at least one copy of the carrier sequence; in some embodiments, rolling circle replication is performed to obtain a DNB formed by a concatemer of the carrier sequence.

As used herein, "DNB" (DNA nano ball) is a typical product of RCA (rolling circle amplification) and possesses the characteristics of RCA products. The RCA product is a multi-copy single-stranded DNA sequence that can form a "spherical" structure due to interactions between bases within the DNA sequence. Typically, library molecules are circularized to form single-stranded circular DNA, which is then amplified by multiple orders of magnitude using rolling circle amplification technology, and the resulting amplification product is referred to as a DNB.

In some embodiments, the carrier sequence further comprises a complementary sequence to the second universal sequence or portion sequence thereof (e.g., the carrier sequence further comprises a complementary sequence to the second universal sequence or 3' end portion sequence thereof), wherein the second universal sequence is as defined above.

In some embodiments, the complementary sequence to the second universal sequence or portion sequence thereof is located 3' of the complementary sequence to the positioning sequence.

In some embodiments, the immobilization primer comprises the second universal sequence or portion sequence thereof (e.g., the immobilization primer comprises the second universal sequence or 5' end portion sequence thereof).

In some embodiments, the extension product comprises, in the direction from 5' to 3': the second universal sequence, the positioning sequence, and the first universal sequence.

In some embodiments, the carrier sequence does not comprise, or further comprises, a template sequence for an MID sequence.

In some embodiments, the carrier sequence further comprises a template sequence for an MID sequence. In some embodiments, the template sequence for the MID sequence is located 3' of the complementary sequence to the first universal sequence, and/or the template sequence for the MID sequence is located 5' of the complementary sequence to the second universal sequence or portion sequence thereof. In some embodiments, the MID sequence consists of 5 to 50 (e.g., 5 to 25, 5 to 35, 5 to 45, 10 to 25, 10 to 35, 10 to 45, 15 to 25, 15 to 35, 15 to 45, 20 to 25, 20 to 35, or 20 to 45) degenerate deoxyribonucleotide residues.

In some embodiments, the immobilization primer is covalently or non-covalently attached to the solid support.

In some embodiments, the immobilization primer is covalently attached to the solid support.

In some embodiments, the immobilization primer is attached to the solid support via a click chemistry reaction.

In some embodiments, the molecular or group pair capable of undergoing the click chemistry reaction is selected from the group consisting of: alkynyl/azido, azido/cyano, amine/ene, thiol/ene, thiol/alkynyl, aldehyde/1,3-diol, ketone/1,3-diol. In some embodiments, the molecule or group pair capable of undergoing the click chemistry reaction is azide/alkynyl.

In some embodiments, the immobilization primer is modified with 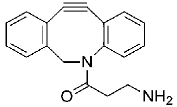 (DBCO), the solid support is surface-modified with azido, and the immobilization primer is attached to the solid support via a click chemistry reaction between DBCO and azido.

In some embodiments, the capture probe is present in a single-stranded form comprising a single-stranded region that comprises a capture sequence, wherein the capture sequence is as defined above, and the (B) comprises:
(1) providing: (a) the capture probe in free form, wherein the capture probe is modified with a molecule or group A'; and (b) the solid support, wherein the solid support is surface-modified with a molecule or group B', and the molecule or group A' is capable of forming a linkage (e.g., a covalent or non-covalent linkage) with the molecule or group B'; and
(2) contacting the capture probe with the solid support under a condition suitable for forming the linkage between the molecule or group A' and the molecule or group B', thereby obtaining the solid support with the capture probe attached thereto.

It is readily understood by those skilled in the art that any part of the capture probe can be modified with the molecule or group A', as long as it does not affect the ability of the capture probe to perform its function (e.g., annealing to a target sequence and initiating an extension reaction from the 3' end). In some embodiments, the capture sequence of the capture probe is not modified with the molecule or group A'. In some embodiments, the 5' end (e.g., the 5' terminus) of the capture probe is modified with the molecule or group A'.

In some embodiments, the capture sequence is located at the 3' end of the capture probe.

In some embodiments, the capture probe is present in a duplex form comprising a single-stranded region that comprises a capture sequence, wherein the capture sequence is as defined above, and wherein the (B) comprises:
(I)
   (1) providing: (a) a free first strand of the capture probe, wherein the first strand is modified with a molecule or group A'; and (b) the solid support, wherein the solid support is surface-modified with a molecule or group B', and the molecule or group A' is capable of forming a linkage (e.g., covalent and/or non-covalent linkage) with the molecule or group B'; and (c) a free second strand of the capture probe, wherein the second strand comprises the capture sequence and is capable of annealing to the first strand to form a duplex;
   (2) contacting the first strand with the solid support under a condition suitable for forming a linkage between the molecule or group A' and the molecule or group B', thereby obtaining a solid support having the first strand attached thereto; and
   (3) contacting the free second strand with the solid support having the first strand attached thereto from step (2) under a condition allowing annealing, thereby obtaining a solid support with the capture probe attached thereto;
   or,
(II)
   (1) providing: (a) a duplex formed by annealing a first strand and a second strand of the capture probe, wherein the first strand is modified with a molecule or group A', and the second strand comprises the capture sequence; and (b) a solid support, wherein the solid support is surface-modified with a molecule or group B', the molecule or group A' is capable of forming a linkage (e.g., covalent and/or non-covalent linkage) with the molecule or group B'; and
   (2) contacting the duplex with the solid support under a condition suitable for forming a linkage between the molecule or group A' and the molecule or group B', thereby obtaining a solid support with the capture probe attached thereto.

In some embodiments, any part of the first strand can be used for attachment to the solid support, as long as such attachment does not affect the ability of the first strand to perform its function (e.g., the function of annealing to the second strand). In some embodiments, the part of the first strand that is not annealed to the second strand is modified with the molecule or group A'. In some embodiments, the terminus (e.g., the 5' terminus or the 3' terminus) of the first strand is modified with the molecule or group A'.

In some embodiments, the second strand is not directly attached to the solid support.

In some embodiments, in the duplex formed by annealing the first strand and the second strand of the capture probe, the second strand comprises the capture sequence at its 3' end, and the capture sequence is not annealed to the first strand.

In some embodiments, the molecule or group A' is capable of undergoing a click chemistry reaction with the molecule or group B'.

In some embodiments, the molecule or group pair A'/B' is selected from the group consisting of: alkynyl/azido, azido/cyano, amine/ene, thiol/ene, thiol/alkynyl, aldehyde/1,3-diol, ketone/1,3-diol, azido/alkynyl, cyano/azido, ene/amine, ene/thiol, alkynyl/thiol, 1,3-diol/aldehyde, 1,3-diol/ketone.

In some embodiments, the molecule or group pair A'/B' is azido/alkynyl or alkynyl/azido.

In some embodiments, the molecule or group A' is DBCO, and the molecule or group B' is azido.

In some embodiments, the first strand of the capture probe is modified with 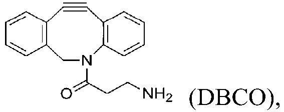 the solid support is surface-modified with azido, and the first strand of the capture probe is attached to the solid support by a click chemistry reaction between DBCO and the azido.

In some embodiments, the solid support has one or more features selected from the following:
(1) the solid support is selected from the group consisting of latex bead, dextran bead, polystyrene surface, polypropylene surface, polyacrylamide gel, gold surface, glass surface, chip, sensor, electrode, and silicon wafer; in some embodiments, the solid support is a chip;
(2) the solid support is planar, spherical, or porous;
(3) the solid support is capable of being used in a sequencing platform; in some embodiments, the solid support is a sequencing chip for Illumina, MGI, or Thermo Fisher sequencing platform; and
(4) the solid support is capable of releasing the positioning probe and/or capture probe spontaneously or upon exposure to one or more stimuli (e.g., temperature change, pH change, exposure to a specific chemical or phase, exposure to light, reducing agent, etc.).

In some embodiments, the same type of positioning probes (i.e., positioning probes comprising the same positioning sequence) occupies the same region on the surface of the solid support, and different types of positioning probes occupy different regions on the surface of the support, and the center-to-center distance between any two adjacent regions is less than 1 µm (e.g., less than 900 nm, less than 800 nm, less than 700 nm, less than 600 nm, less than 500 nm, less than 400 nm, less than 300 nm, less than 200 nm).

In some embodiments, the capture probes and the positioning probes are distributed on the solid support in such a way that nucleic acid molecules linked to the capture probes (e.g., nucleic acid molecules annealed to the capture probes, or nucleic acid molecules obtained by extension of the capture probes through a nucleic acid polymerization reaction) can contact their adjacent positioning probes.

In some embodiments, there are at least 100 capture probes that are distributed in proximity to each type of positioning probe. For example, there are at least 100, at least 200, at least 300, at least 400, at least 500, at least 600, at least 700, at least 800, at least 900, at least 1000, at least 1500, at least 2000, at least 2500, at least 3000, at least 3500, at least 4000, at least 4500, at least 5000, at least 5500, at least 6000, at least 6500, at least 7000, at least 7500, at least 8000, at least 8500, at least 9000, at least 9500, or at least 10000 of the described capture probes.

In some embodiments, there are 500 to 10000 (e.g., 1000 to 1500, 1000 to 2000, 1000 to 3000, 1000 to 4000, 1000 to 5000, 1000 to 8000, or 1000 to 10000) of the capture probes that are distributed in proximity to each type of the positioning probes.

In some embodiments, the expression "in proximity to each type of the positioning probes" refers to a region on the surface of the solid support adjacent to the type of positioning probes, wherein nucleic acid molecules linked to the capture probes distributed in the region (e.g., nucleic acid molecules annealed to the capture probes, or nucleic acid molecules obtained by extension of the capture probes via a nucleic acid polymerization reaction) are capable of contacting the type of positioning probes.

In some embodiments, at least 100 of the capture probes are distributed around the center of the location occupied by each type of the positioning probes on the solid support (e.g., the center of the area occupied by each type of the positioning probes on the surface of the solid support) within a radius of 200 nm to 300 nm. For example, there are at least 100, at least 200, at least 300, at least 400, at least 500, at least 600, at least 700, at least 800, at least 900, at least 1000, at least 1500, at least 2000, at least 2500, at least 3000, at least 3500, at least 4000, at least 4500, at least 5000, at least 5500, at least 6000, at least 6500, at least 7000, at least 7500, at least 8000, at least 8500, at least 9000, at least 9500, or at least 10000 of the described capture probes.

In some embodiments, 500 to 10000 (e.g., 1000 to 1500, 1000 to 2000, 1000 to 3000, 1000 to 4000, 1000 to 5000, 1000 to 8000, 1000 to 10000) of the capture probes are distributed within a radius of 200 to 300 nm around the center of the location occupied by each type of the positioning probes on the solid support (e.g., the center of the area occupied by each type of the positioning probes on the surface of the solid support).

### Preparation method of nucleic acid array

In another aspect, the present application provides a method for preparing the nucleic acid array as described above, which comprises the following:
(A) providing a nucleic acid array to be processed, wherein the nucleic acid array to be processed comprises a solid support, the solid support is attached with at least two types of oligonucleotide molecules;
   each type of oligonucleotide molecule occupies a different position on the solid support;
   the oligonucleotide molecule comprises, in the direction from 5' to 3', a positioning sequence and a first universal sequence sequentially, wherein the positioning sequence has a nucleotide sequence corresponding to the position of the type of oligonucleotide molecule on the solid support; the first universal sequence is as defined above;
   and,
(B) attaching and/or synthesizing a capture probe on the solid support comprised in the nucleic acid array to be processed, wherein the capture probe is as defined above.

In some embodiments, each type of oligonucleotide molecule consists of one or more oligonucleotide molecules.

In some embodiments, the positioning sequences comprised in the oligonucleotide molecules of the same type are identical to each other, while the positioning sequences comprised in the oligonucleotide molecules of different types are different from each other.

It will be readily appreciated by those skilled in the art that one or more oligonucleotide molecules of the oligonucleotide molecules of the same type share the same positioning sequence; however, it is not required that every oligonucleotide molecule of each type of oligonucleotide molecule have the same complete sequence.

In some embodiments, the oligonucleotide molecule does not comprise a capture sequence located 3' of the first universal sequence, wherein the capture sequence is as defined above.

In some embodiments, the oligonucleotide molecule comprises a capture sequence located 3' of the first universal sequence, wherein the capture sequence is as defined above, and the method further comprises (B'): removing the capture sequence comprised in the oligonucleotide molecule;
wherein the (B) and the (B') can be performed in any order or simultaneously (e.g., performed simultaneously in the same reaction system).

In some embodiments, in the (B'), the capture sequence comprised in the oligonucleotide molecule is removed by steps comprising:
(i) providing a blocking probe, wherein the blocking probe is capable of annealing to (a) the first universal sequence of the oligonucleotide molecule or a portion sequence thereof (e.g., a 3' end portion sequence of the first universal sequence), or (b) a sequence in the oligonucleotide molecule, which is located 5' of the capture sequence and 3' of the first universal sequence, or (c) a combination of (a) and (b);
(ii) contacting the blocking probe with the nucleic acid array to be processed that comprises the oligonucleotide molecule under a condition suitable for annealing the blocking probe to the oligonucleotide molecule;
(iii) contacting an exonuclease with the product of step (ii) under a condition that allows the exonuclease to exert its cleavage activity; wherein the exonuclease has single-stranded nucleic acid-specific 3'-to-5' exonucleolytic activity.

In some embodiments, the exonuclease is exonuclease I.

In some embodiments, the (B') further comprises a step of removing the blocking probe (e.g., by melting the blocking probe away from the oligonucleotide molecule, thereby removing the blocking probe).

In some embodiments, in the described method, the (B) is performed after the (B').

In some embodiments, the oligonucleotide molecule further comprises a second universal sequence, wherein the second universal sequence is as defined above.

In some embodiments, the second universal sequence is located 5' of the positioning sequence.

In some embodiments, the oligonucleotide molecule does not comprise or further comprises an MID sequence.

In some embodiments, the oligonucleotide molecule further comprises an MID sequence. In some embodiments, the MID sequence is located 5' of the first universal sequence, and/or the MID sequence is located 3' of the second universal sequence. In some embodiments, the MID sequences comprised in the same type of oligonucleotide molecule are different from each other.

In some embodiments, in the nucleic acid array to be processed, the oligonucleotide molecule is covalently or non-covalently attached to the solid support.

In some embodiments, the oligonucleotide molecule is covalently attached to the solid support.

In some embodiments, the oligonucleotide molecule is attached to the solid support via a click chemistry reaction.

In some embodiments, the molecule or group pair capable of undergoing the click chemistry reaction is selected from the group consisting of: alkynyl/azido, azido/cyano, amine/ene, thiol/ene, thiol/alkynyl, aldehyde/1,3-diol, ketone/1,3-diol. In some embodiments, the molecule or group pair capable of undergoing the click chemistry reaction is azido/alkynyl.

In some embodiments, the solid support is surface-modified with azido, the oligonucleotide molecule is modified with 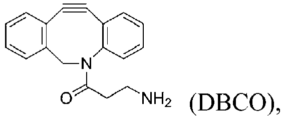 and the oligonucleotide molecule is attached to the solid support via a click chemistry reaction between the azido and the DBCO.

In some embodiments, the capture probe is present in a single-stranded form comprising a single-stranded region that comprises a capture sequence, wherein the capture sequence is as defined above, and the (B) comprises:
(1) providing: (a) the capture probe in the free form, wherein the capture probe is modified with a molecule or group A'; and (b) the nucleic acid array to be processed or the nucleic acid array processed in the (B'), wherein the solid support of the nucleic acid array is surface-modified with a molecule or group B', and the molecule or group A' is capable of forming a linkage (e.g., a covalent or non-covalent linkage) with the molecule or group B'; and,
(2) contacting the capture probe with the solid support under a condition suitable for forming a linkage between the molecule or group A' and the molecule or group B', thereby obtaining a solid support with the capture probe attached thereto.

It will be readily appreciated by those skilled in the art that any part of the capture probe can be used for the modification with the molecule or group A', as long as it does not affect the function of the capture probe (e.g., the function of annealing to the target sequence and initiating the extension reaction from the 3' end). In some embodiments, the capture sequence of the capture probe is not modified with the molecule or group A'. In some embodiments, the 5' end (e.g., the 5' terminus) of the capture probe is modified with the molecule or group A'.

In some embodiments, the capture sequence is located at the 3' end of the capture probe.

In some embodiments, the capture probe is present in a duplex form comprising a single-stranded region that comprises a capture sequence, wherein the capture sequence is as defined above, and the (B) comprises:
(I)
   (1) providing: (a) a free first strand of the capture probe, wherein the first strand is modified with a molecule or group A'; and (b) the nucleic acid array to be processed or the nucleic acid array processed in the (B'), wherein the solid support of the nucleic acid array is surface-modified with a molecule or group B', and the molecule or group A' is capable of forming a linkage (e.g., covalent and/or non-covalent linkage) with the molecule or group B'; and (c) a free second strand of the capture probe, wherein the second strand comprises the capture sequence and is capable of annealing to the first strand to form a duplex;
   (2) contacting the first strand with the solid support under a condition suitable for forming a linkage between the molecule or group A' and the molecule or group B', thereby obtaining a solid support with the first strand attached thereto; and
   (3) contacting the free second strand with the solid support with the first strand attached thereto obtained in step (2) under a condition that allows annealing, thereby obtaining a solid support with the capture probe attached thereto;
   or,
(II)
   (1) providing: (a) a duplex formed by annealing a first strand and a second strand of the capture probe, wherein the first strand is modified with a molecule or group A', and the second strand comprises the capture sequence; and (b) the nucleic acid array to be processed or the nucleic acid array processed in the (B'), wherein the solid support of the nucleic acid array is surface-modified with a molecule or group B', and the molecule or group A' is capable of forming a linkage (e.g., covalent and/or non-covalent linkage) with the molecule or group B'; and
   (2) contacting the duplex with the solid support under a condition suitable for forming a linkage between the molecule or group A' and the molecule or group B', thereby obtaining a solid support with the capture probe attached thereto.

In some embodiments, any part of the first strand can be used for the attachment to the solid support, as long as such linkage does not affect the function of the first strand (e.g., the function of annealing to the second strand). In some embodiments, the part of the first strand that does not anneal to the second strand is modified with the molecule or group A'. In some embodiments, the terminus (e.g., the 5' terminus or the 3' terminus) of the first strand is modified with the molecule or group A'.

In certain embodiments, the second strand is not directly attached to the solid support.

In certain embodiments, in the duplex formed by annealing the first strand and the second strand of the capture probe, the second strand comprises the capture sequence at its 3' end, and the capture sequence does not anneal to the first strand.

In some embodiments, the molecule or group A' is capable of undergoing a click chemistry reaction with the molecule or group B'.

In some embodiments, the molecule or group pair A'/B' is selected from the group consisting of: alkynyl/azido, azido/cyano, amine/ene, thiol/ene, thiol/alkynyl, aldehyde/1,3-diol, ketone/1,3-diol, azido/alkynyl, cyano/azido, ene/amine, ene/thiol, alkynyl/thiol, 1,3-diol/aldehyde, 1,3-diol/ketone.

In some embodiments, the molecule or group pair A'/B' is azido/alkynyl or alkynyl/azido.

In some embodiments, the molecule or group A' is DBCO, and the molecule or group B' is azido.

In some embodiments, the first strand of the capture probe is modified with 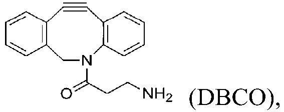 the solid support is surface-modified with azido, and the first strand of the capture probe is attached to the solid support by a click chemistry reaction between DBCO and azido.

In some embodiments, the solid support has one or more features selected from the following:
(1) the solid support is selected from the group consisting of latex bead, dextran bead, polystyrene surface, polypropylene surface, polyacrylamide gel, gold surface, glass surface, chip, sensor, electrode, and silicon wafer; in some embodiments, the solid support is a chip;
(2) the solid support is planar, spherical, or porous;
(3) the solid support is capable of being used in a sequencing platform; in some embodiments, the solid support is a sequencing chip for use in an Illumina, MGI, or Thermo Fisher sequencing platform; and
(4) the solid support is capable of releasing the positioning probe and/or capture probe spontaneously or upon exposure to one or more stimuli (e.g., temperature change, pH change, exposure to a specific chemical or phase, exposure to light, reducing agent, etc.).

In some embodiments, positioning probes of the same type (i.e., positioning probes comprising the same positioning sequence) occupy the same region on the surface of the solid support, and positioning probes of different types occupy different regions on the surface of the support, and the center-to-center distance between any two adjacent regions is less than 1 µm (e.g., less than 900 nm, less than 800 nm, less than 700 nm, less than 600 nm, less than 500 nm, less than 400 nm, less than 300 nm, less than 200 nm).

In some embodiments, the capture probes and the positioning probes are distributed on the solid support in such a way that a nucleic acid molecule linked to the capture probe (e.g., a nucleic acid molecule annealed to the capture probe, or a nucleic acid molecule obtained by extension of the capture probe through a nucleic acid polymerization reaction) can contact a positioning probe in proximity thereto.

In some embodiments, at least 100 of the capture probes are distributed in proximity to each type of the positioning probes. For example, there are at least 100, at least 200, at least 300, at least 400, at least 500, at least 600, at least 700, at least 800, at least 900, at least 1000, at least 1500, at least 2000, at least 2500, at least 3000, at least 3500, at least 4000, at least 4500, at least 5000, at least 5500, at least 6000, at least 6500, at least 7000, at least 7500, at least 8000, at least 8500, at least 9000, at least 9500, or at least 10000 of the described capture probes.

In some embodiments, there are 500 to 10000 (e.g., 1000 to 1500, 1000 to 2000, 1000 to 3000, 1000 to 4000, 1000 to 5000, 1000 to 8000, or 1000 to 10000) of the capture probes that are distributed in proximity to each type of the positioning probes.

In some embodiments, the expression "in proximity to each type of the positioning probes" refers to a region on the surface of the solid support adjacent to the type of positioning probes, wherein nucleic acid molecules linked to the capture probes distributed in the region (e.g., nucleic acid molecules annealed to the capture probes, or nucleic acid molecules obtained by extension of the capture probes via a nucleic acid polymerization reaction) are capable of contacting the type of positioning probes.

In some embodiments, at least 100 of the capture probes are distributed around the center of the location occupied by each type of the positioning probes on the solid support (e.g., the center of the area occupied by each type of the positioning probes on the surface of the solid support) within a radius of 200 nm to 300 nm. For example, there are at least 100, at least 200, at least 300, at least 400, at least 500, at least 600, at least 700, at least 800, at least 900, at least 1000, at least 1500, at least 2000, at least 2500, at least 3000, at least 3500, at least 4000, at least 4500, at least 5000, at least 5500, at least 6000, at least 6500, at least 7000, at least 7500, at least 8000, at least 8500, at least 9000, at least 9500, or at least 10000 of the described capture probes.

In some embodiments, 500 to 10000 (e.g., 1000 to 1500, 1000 to 2000, 1000 to 3000, 1000 to 4000, 1000 to 5000, 1000 to 8000, 1000 to 10000) of the capture probes are distributed within a radius of 200 to 300 nm around the center of the location occupied by each type of the positioning probes on the solid support (e.g., the center of the area occupied by each type of the positioning probes on the surface of the solid support).

### Method for detecting spatial information of nucleic acid

In another aspect, the present application provides a method for detecting the spatial information of a nucleic acid in a sample, which comprises the following steps:
(1) providing a nucleic acid array as described above;
(2) contacting the nucleic acid array with a sample to be tested under a condition allowing annealing, such that a nucleic acid derived from the sample to be tested anneals to a capture sequence in a capture probe of the nucleic acid array;
(3) performing a nucleic acid polymerization reaction under a condition allowing nucleic acid polymerization to generate a first extension product, wherein the first extension product comprises the capture sequence and a complementary sequence to a nucleic acid molecule or portion sequence thereof capable of annealing to the capture sequence, and the first extension product is attached via the capture sequence comprised therein to the solid support;
(4) annealing the first extension product attached to the solid support to a positioning probe in proximity thereto on the nucleic acid array under a condition allowing annealing;
   wherein, a first universal sequence of the positioning probe is capable of annealing to the first extension product; and the first universal sequence is located 3' of the positioning sequence of the positioning probe;
(5) performing a nucleic acid polymerization reaction under a condition allowing nucleic acid polymerization to generate a second extension product and/or a complementary strand thereof, wherein the second extension product comprises: (a) the positioning sequence of the positioning probe and a complementary sequence to the first extension product or portion sequence thereof, or (b) the sequence of the first extension product or portion sequence thereof, and a complementary sequence to the positioning sequence of the positioning probe; the positioning sequence or complementary sequence thereof comprised in the second extension product serves as its spatial information tag, and the positioning sequence or complementary sequence thereof comprised in the complementary strand to the second extension product serves as its spatial information tag, so that the position of the nucleic acid derived from the sample to be tested in the sample to be tested is mapped to the position of the positioning probe corresponding to the positioning sequence; thereby obtaining a nucleic acid molecule comprising a spatial information tag; and
(6) analyzing the sequence of: (i) the nucleic acid molecule comprising the spatial information tag obtained in step (5), and/or (ii) a nucleic acid molecule derived from the nucleic acid molecule comprising the spatial information tag of (i).

It is easily understood by those skilled in the art that, in steps (4) to (5), the first extension product anneals to the positioning probe in proximity thereto and undergoes a nucleic acid polymerization reaction to generate the second extension product, whereby the second extension product comprises the positioning sequence of the positioning probe or a complementary sequence thereof, thereby mapping the position of the nucleic acid derived from the sample to be tested captured by the capture probe to the position of the positioning probe annealed to the first extension product on the nucleic acid array.

In some embodiments, the spatial information of the nucleic acid comprises the localization, distribution, and/or abundance of the nucleic acid.

In some embodiments, in step (3), the nucleic acid molecule annealed to the capture sequence is used as a template in the nucleic acid polymerization reaction to extend the capture sequence.

In some embodiments, the capture sequence is located at the 3' end of the capture probe, and/or the capture sequence has a free hydroxyl (-OH) at the 3' end.

In certain embodiments, in step (5), the first extension product is used as a template in the polymerization reaction to extend the positioning probe; and/or the positioning probe is used as a template to extend the first extension product.

In some embodiments, in step (5), the first extension product is used as a template in the polymerization reaction to extend the positioning probe, so as to generate the second extension product and/or a complementary strand thereof, the second extension product comprising the positioning sequence of the positioning probe and a complementary sequence to the first extension product or portion sequence thereof. In some embodiments, the first universal sequence of the positioning probe is located at the 3' end of the positioning probe, and/or, the first universal sequence of the positioning probe has a free hydroxyl (-OH) at the 3' end.

In some embodiments, in step (5), the positioning probe is used as a template in the polymerization reaction to extend the first extension product, so as to generate the second extension product and/or a complementary strand thereof, the second extension product comprising the sequence of the first extension product or portion sequence thereof and a complementary sequence to the positioning sequence of the positioning probe. In some embodiments, the first universal sequence of the positioning probe may or may not be located at the 3' end of the positioning probe, and/or, the 3' end of the first universal sequence of the positioning probe is blocked or unblocked.

In some embodiments, in step (5), during the polymerization reaction, the first extension product and the positioning probe are used as templates for each other to extend the first extension product and the positioning probe, so as to generate the second extension product and/or a complementary strand thereof. The second extension product comprises the positioning sequence of the positioning probe and a complementary sequence to the first extension product or portion sequence thereof, and the second extension product comprises the sequence of the first extension product or portion sequence thereof and a complementary sequence to the positioning sequence of the positioning probe. In some embodiments, the first universal sequence of the positioning probe is located at the 3' end of the positioning probe, and/or the first universal sequence of the positioning probe has a free hydroxyl (-OH) at the 3' end.

In some embodiments, in step (6), analysis is performed on the sequence of the nucleic acid molecule that is attached to the solid support of the nucleic acid array and comprises the spatial information tag.

In some embodiments, before step (6) and after step (5), the method further comprises step pre-(6): releasing at least a part of the nucleic acid molecules comprising the spatial information tag from the surface of the nucleic acid array.

In some embodiments, in step (6), analysis is performed on the sequence of (i) the nucleic acid molecule comprising the spatial information tag released in step pre-(6), and/or, (ii) a nucleic acid molecule derived from the nucleic acid molecule of (i) and comprising the spatial information tag.

In some embodiments, in step pre-(6), the nucleic acid molecule is released from the surface of the solid support by the following method: (i) nucleic acid cleavage; and/or, (ii) denaturation.

In some embodiments, after step pre-(6) and before step (6), the method further comprises a step of amplifying the released nucleic acid molecule.

In some embodiments, before performing step (6), the method further comprises a step of purifying the released nucleic acid molecule.

In some embodiments, the method comprises one or more features selected from the following:
(i) in step (1), the nucleic acid array is provided by the method for preparing nucleic acid array as described above;
(ii) in step (2), the sample to be tested is processed (e.g., by permeabilization or cell lysis) to release a nucleic acid derived from the sample to be tested, and allow it to anneal to the capture sequence;
(iii) after step (3) and before step (4), the method further comprises a step of removing a template strand bound to the first extension product (e.g., removing a template strand bound to the first extension product by enzyme digestion or melting);
(iv) after step (3) and before step (4), the method further comprises a step of washing the nucleic acid array to remove residual sample (e.g., tissue or cells);
(v) after step (5) and before step (6) (e.g., after step (5) and before step pre-(6)), the method further comprises a step of amplifying (e.g., in-situ amplifying) the nucleic acid molecule comprising the spatial information tag. In some embodiments, the nucleic acid molecule comprising the spatial information tag is attached to the solid support of the nucleic acid array.

In some embodiments, the sample is a tissue sample (e.g., a tissue section) or a single-cell sample (e.g., a single-cell suspension).

In some embodiments, the tissue sample (e.g., tissue section) is prepared from fixed tissue, such as a formalin-fixed paraffin-embedded (FFPE) tissue, a deep-frozen tissue, or a fresh tissue.

In some embodiments, the cell is an immune cell, such as a B cell or a T cell. In some embodiments, the nucleic acid derived from the sample to be tested comprises a T cell receptor gene or nucleic acid product (e.g., mRNA) thereof, or a B cell receptor gene or nucleic acid product (e.g., mRNA) thereof.

In some embodiments, the nucleic acid derived from the sample to be tested is selected from the group consisting of: an RNA molecule (e.g., an mRNA), a target nucleic acid molecule (e.g., a target DNA and/or RNA), a genomic nucleic acid fragment located in open region of chromatin, and any combination thereof.

In some embodiments, in step (6), the analysis comprises sequencing and/or sequence-specific PCR reaction.

In some embodiments, prior to sequencing, the method further comprises a step of constructing a sequencing library from the nucleic acid molecule comprising the spatial information tag obtained in step (5) or an amplification product thereof.

In some embodiments, the method is used for detecting the spatial information of an RNA (e.g., an mRNA) in cells of a sample.

In some embodiments, in step (2), the nucleic acid derived from the sample to be tested is an RNA (e.g., an mRNA) from a cell of the sample to be tested, and the capture sequence comprises a poly(dT) sequence or a random oligonucleotide sequence.

In some embodiments, the step (3) comprises:
(a) under a condition allowing nucleic acid polymerization, using a nucleic acid molecule annealed to the capture sequence as a template, performing a nucleic acid polymerization reaction to extend the capture sequence, so as to generate a cDNA strand, wherein the cDNA strand comprises a cDNA sequence that is generated by a reverse transcription primed by the capture sequence and is complementary to the RNA (e.g., mRNA), and a 3' end overhang;
(b) under a condition allowing nucleic acid polymerization, annealing a template-switching sequence to the cDNA strand generated in (a), and continuing the nucleic acid polymerization reaction using the template-switching sequence as a template to generate the first extension product;

wherein the template-switching sequence comprises a common sequence, a complementary sequence to the 3' end overhang, and optionally an MID sequence; in some embodiments, the MID sequence comprised in each template-switching sequence is different from one another;
the first extension product comprises: the capture sequence, the cDNA strand sequence or portion sequence thereof, and a complementary sequence to the common sequence.

In some embodiments, the 3' end overhang has a length of at least 1, at least 2, at least 3, at least 4, at least 5, at least 6, at least 7, at least 8, at least 9, at least 10, 1 to 10, 1 to 5, or 2 to 10 nucleotides. In some embodiments, the 3' end overhang is an overhang consisting of 2 to 5 cytidine nucleotides (e.g., a CCC overhang).

In some embodiments, the method further includes, prior to step (4), a step of removing the template-switching sequence bound to the first extension product (e.g., removing the template-switching sequence bound to the first extension product by enzyme digestion or melting).

In some embodiments, in step (4) of the method, the first universal sequence of the positioning probe is capable of annealing to a complementary sequence to the common sequence.

In some embodiments, the positioning probe does not comprise an MID sequence, and the template-switching sequence comprises an MID sequence; or, the positioning probe comprises an MID sequence, and the template-switching sequence does not comprise an MID sequence; or, both the positioning probe and the template-switching sequence comprise MID sequences.

In some embodiments, the method is used for detecting spatial information of a target nucleic acid (e.g., a target DNA and/or RNA) of cells in a sample, and the target nucleic acid comprises a target nucleotide sequence.

In some embodiments, in step (2), the nucleic acid derived from the sample to be tested comprises a target nucleotide sequence. In some embodiments, the nucleic acid derived from the sample to be tested comprises the target nucleic acid and/or a nucleic acid molecule derived from the target nucleic acid, and the nucleic acid molecule derived from the target nucleic acid comprises the target nucleotide sequence.

In some embodiments, in step (2), the capture sequence comprises a sequence capable of specifically recognizing the target nucleotide sequence.

In some embodiments, in step (2), the capture sequence comprises a sequence capable of specifically recognizing a first segment of the target nucleotide sequence; in step (4), the first universal sequence of the positioning probe comprises a sequence capable of specifically recognizing a complementary sequence to a second segment of the target nucleotide sequence, or the first universal sequence comprises a random oligonucleotide sequence; in some embodiments, in the target nucleotide sequence, the first segment is located 3' of the second segment.

In some embodiments, in step (2), the first segment is present in a single-stranded region of a nucleic acid molecule that is derived from the sample to be tested and annealed to the capture sequence.

In some embodiments, in step (2), the nucleic acid that is derived from the sample to be tested and annealed to the capture sequence is a single-stranded or double-stranded nucleic acid comprising a single-stranded region that comprises the first segment.

In some embodiments, the method is used for detecting spatial information of an RNA (e.g., an mRNA) and a target nucleic acid (e.g., a target DNA and/or RNA) of cells in a sample, wherein the target nucleic acid comprises a target nucleotide sequence.

In some embodiments, the nucleic acid array comprises a first capture probe capable of capturing an RNA (e.g., an mRNA), and a second capture probe capable of capturing a nucleic acid molecule comprising a target nucleotide sequence; the first capture probe comprises a first capture sequence, the first capture sequence comprising a poly(dT) sequence or a random oligonucleotide sequence; the second capture probe comprises a second capture sequence, the second capture sequence comprising a sequence capable of specifically recognizing the target nucleotide sequence.

It will be readily understood by those skilled in the art that, unless otherwise specified herein or clearly contradicted by the context, the definitions/descriptions of the capture probe above also apply to the first capture probe and the second capture probe, and the definitions/descriptions of the capture sequence above also apply to the first capture sequence and the second capture sequence.

In some embodiments, in step (2), the nucleic acid derived from the sample to be tested comprises an RNA (e.g., an mRNA) derived from the sample to be tested and a nucleic acid molecule comprising the target nucleotide sequence; in some embodiments, the nucleic acid molecule comprises the target nucleotide sequence comprises the target nucleic acid and/or a nucleic acid molecule derived from the target nucleic acid.

In some embodiments, the step (3) comprises:
(A)
   (a) under a condition allowing nucleic acid polymerization, using a nucleic acid molecule annealed to the first capture sequence as a template, performing a nucleic acid polymerization reaction to extend the first capture sequence, so as to generate a cDNA strand, wherein the cDNA strand comprises a cDNA sequence that is generated by a reverse transcription primed by the first capture sequence and is complementary to the RNA (e.g., mRNA), and a 3' end overhang;
   (b) under a condition allowing nucleic acid polymerization, annealing a template-switching sequence to the cDNA strand generated in (a), and continuing the nucleic acid polymerization reaction using the template-switching sequence as a template to generate a first extension product I;
   wherein, the template-switching sequence comprises a common sequence, a complementary sequence to the 3' end overhang, and optionally an MID sequence; in some embodiments, the MID sequence comprised in each template-switching sequence is different from each other;
   and,
(B) under a condition allowing nucleic acid polymerization, using a nucleic acid molecule annealed to the second capture sequence as a template, performing a nucleic acid polymerization reaction to extend the second capture sequence, so as to generate a first extension product II, wherein the first extension product II comprises the second capture sequence and a complementary sequence to the nucleic acid molecule or portion sequence thereof annealed to the second capture sequence;
wherein, the (B) and the (A) are performed in any order; for example, the (B) is performed before or after the (A), or the (B) is performed simultaneously with the (A) (e.g., in the same reaction system).

In some embodiments, the 3' end overhang has a length of at least 1, at least 2, at least 3, at least 4, at least 5, at least 6, at least 7, at least 8, at least 9, at least 10, 1 to 10, 1 to 5, or 2 to 10 nucleotides. In some embodiments, the 3' end overhang is an overhang consisting of 2 to 5 cytosine nucleotides (e.g., a CCC overhang).

In some embodiments, in step (4), the positioning probe comprises a first universal sequence I capable of annealing to the first extension product I, and a first universal sequence II capable of annealing to the first extension product II; in some embodiments, the first universal sequence I and the first universal sequence II coexist on the same positioning probe, or are present on different positioning probes, respectively.

In some embodiments, the nucleic acid array comprises a first positioning probe that comprises the first universal sequence I, and a second positioning probe that comprises the first universal sequence II.

It will be readily understood by those skilled in the art that, unless otherwise specified herein or clearly contradicted by the context, the definitions/descriptions of the positioning probe above also apply to the first positioning probe and the second positioning probe, and the definitions/descriptions of the first universal sequence above also apply to the first universal sequence I and the first universal sequence II.

In some embodiments, in step (4) of the method, the first universal sequence I is capable of annealing to a complementary sequence to the common sequence.

In some embodiments, in step (2) of the method, the second capture sequence comprises a sequence capable of specifically recognizing a first segment of the target nucleotide sequence; in step (4), the first universal sequence II comprises a sequence capable of specifically recognizing a complementary sequence to a second segment of the target nucleotide sequence, or the first universal sequence II comprises a random oligonucleotide sequence. In some embodiments, in the target nucleotide sequence, the first segment is located 3' of the second segment.

In some embodiments, the method further comprises, prior to step (4), a step of removing the template-switching sequence bound to the first extension product I (e.g., removing the template-switching sequence bound to the first extension product I by enzymatic digestion or melting).

In some embodiments, in step (2), the first segment is present in a single-stranded region of a nucleic acid molecule that is derived from the sample to be tested and annealed to the second capture sequence.

In some embodiments, in step (2), the nucleic acid that is derived from the sample to be tested and annealed with the second capture sequence is a single-stranded or double-stranded nucleic acid comprising a single-stranded region that comprises the first segment.

In some embodiments, the first positioning probe does not comprise an MID sequence, and the template-switching sequence comprises an MID sequence; or, the first positioning probe comprises an MID sequence, and the template-switching sequence does not comprise an MID sequence; or, both the first positioning probe and the template-switching sequence comprise MID sequences.

### Kit

In another aspect, the present application provides a kit, which comprises the nucleic acid array as described above.

In some embodiments, the kit further comprises an instruction for use. In some embodiments, the instruction for use describes the method for detecting nucleic acid spatial information as described above.

In some embodiments, the nucleic acid spatial information comprises the localization, distribution, and/or abundance of the nucleic acid.

### For capture of mRNA

In some embodiments, the capture sequence of the nucleic acid array comprises a poly(dT) sequence or a random oligonucleotide sequence.

In some embodiments, the kit further comprises a template-switching sequence, wherein the template-switching sequence comprises a common sequence, a complementary sequence to a 3' end overhang of cDNA, and optionally an MID sequence; in some embodiments, the MID sequence comprised in each template-switching sequence is different from one another. In some embodiments, the 3' end overhang of cDNA has a length of at least 1, at least 2, at least 3, at least 4, at least 5, at least 6, at least 7, at least 8, at least 9, at least 10, 1 to 10, 1 to 5, or 2 to 10 nucleotides. In some embodiments, the 3' end overhang of cDNA is an overhang consisting of 2 to 5 cytosine nucleotides (e.g., a CCC overhang).

In some embodiments, a complementary sequence to the common sequence of the template-switching sequences is capable of annealing to the first universal sequence of the positioning probe of the nucleic acid array.

In some embodiments, the positioning probe does not comprise an MID sequence, and the template-switching sequence comprises an MID sequence; or, the positioning probe comprises an MID sequence, and the template-switching sequence does not comprise an MID sequence; or, both the positioning probe and the template-switching sequence comprises MID sequences.

### For capture of target nucleic acid

In some embodiments, the capture sequence of the nucleic acid array comprises a sequence capable of specifically recognizing a target nucleotide sequence comprised in a target nucleic acid (e.g., a certain target DNA and/or RNA).

In some embodiments, the first universal sequence comprises a sequence capable of specifically recognizing a complementary sequence to the target nucleotide sequence.

In some embodiments, the capture sequence comprises a sequence capable of specifically recognizing a first segment of the target nucleotide sequence, and the first universal sequence of the positioning probe of the nucleic acid array comprises a sequence capable of specifically recognizing a complementary sequence to a second segment of the target nucleotide sequence, or, the first universal sequence comprises a random oligonucleotide sequence.

In some embodiments, in the target nucleotide sequence, the first segment is located 3' of the second segment.

### For dual-capture of mRNA and target nucleic acid

In some embodiments, the nucleic acid array comprises a first capture probe and a second capture probe; the first capture probe comprises a first capture sequence, wherein the first capture sequence comprises a poly(dT) sequence or a random oligonucleotide sequence; the second capture probe comprises a second capture sequence, wherein the second capture sequence comprises a sequence capable of specifically recognizing a target nucleotide sequence comprised in a target nucleic acid (e.g., a certain target DNA and/or RNA).

It will be readily understood by those skilled in the art that, unless otherwise specified herein or clearly contradicted by the context, the definitions/descriptions above for the capture probes also apply to the first capture probe and the second capture probe, and the definitions/descriptions above regarding the capture sequence also apply to the first capture sequence and the second capture sequence.

In some embodiments, the kit further comprises a template-switching sequence, wherein the template-switching sequence comprises a common sequence, a complementary sequence to a 3' end overhang of cDNA, and optionally an MID sequence. In some embodiments, the MID sequence comprised in each template-switching sequence is different from each other. In some embodiments, the 3' end overhang of cDNA has a length of at least 1, at least 2, at least 3, at least 4, at least 5, at least 6, at least 7, at least 8, at least 9, at least 10, 1 to 10, 1 to 5, or 2 to 10 nucleotides. In some embodiments, the 3' end overhang of cDNA is an overhang consisting of 2 to 5 cytosine nucleotides (e.g., a CCC overhang).

In some embodiments, the positioning probe of the nucleic acid array comprises a first universal sequence I and a first universal sequence II; wherein the first universal sequence I is capable of annealing to a complementary sequence to the common sequence, and the first universal sequence II comprises a sequence capable of specifically recognizing a complementary sequence to the target nucleotide sequence, or the first universal sequence II comprises a random oligonucleotide sequence. In some embodiments, the first universal sequence I and the first universal sequence II coexist on the same positioning probe, or they are present on different positioning probes.

In some embodiments, the nucleic acid array comprises a first positioning probe that comprises the first universal sequence I, and a second positioning probe that comprises the first universal sequence II. In some embodiments, the first targeting probe does not comprise an MID sequence, and the template-switching sequence comprises an MID sequence; or, the first targeting probe comprises an MID sequence, and the template-switching sequence does not comprise an MID sequence; or, both the first targeting probe and the template-switching sequence comprise MID sequences.

It will be readily understood by those skilled in the art that, unless otherwise specified herein or clearly contradicted by the context, the definitions/descriptions above regarding the positioning probe also apply to the first positioning probe and the second positioning probe, and the definitions/descriptions above regarding the first universal sequence also apply to the first universal sequence I and the first universal sequence II.

In some embodiments, the second capture sequence comprises a sequence capable of specifically recognizing a first segment of the target nucleotide sequence, and the first universal sequence II comprises a sequence capable of specifically recognizing a complementary sequence to a second segment of the target nucleotide sequence; or, the first universal sequence II comprises a random oligonucleotide sequence. In some embodiments, in the target nucleotide sequence, the first segment is located 3' of the second segment.

In some embodiments, the first universal sequence comprised in the positioning probe of the nucleic acid array is or is not located at the 3' end of the positioning probe, and/or, the 3' end of the first universal sequence of the positioning probe of the nucleic acid array is or is not blocked.

In some embodiments, the kit further comprises: a reagent for nucleic acid hybridization, a reagent for nucleic acid extension, a reagent for nucleic acid amplification, a reagent for recovering or purifying nucleic acid, a reagent for constructing a transcriptome sequencing library, a reagent for sequencing (e.g., second-generation sequencing or third-generation sequencing), or any combination thereof.

On another aspect, the present application provides a use of the nucleic acid array or kit described above for constructing a nucleic acid molecular library, for performing nucleic acid sequencing, or for detecting spatial information of a nucleic acid in a sample.

In some embodiments, the spatial information of the nucleic acid comprises the localization, distribution, and/or abundance of the nucleic acid.

As used herein, "target nucleotide sequence" and "targeted nucleotide sequence" have the same meaning and are used interchangeably.

### Beneficial effects of the invention

In the nucleic acid array (e.g., a capture chip) provided in the present application, the capture region and the spatial information region are present in two independent molecules. For example, the capture region and the spatial information region are present in a capture probe and a positioning probe, respectively. The positioning probe is used to provide spatial location information for spatial omics, while the capture probe is used to capture an intracellular molecule. This eliminates the limitation of the number of capture probes being affected by spatial density, allowing full utilization of all regions on the chip to graft capture probes, thereby improving the capture efficiency of intracellular molecules while maintaining high resolution.

The embodiments of the present invention will be described in detail below with reference to the accompanying drawings and examples. However, those skilled in the art will understand that the following drawings and examples are for illustrative purposes only and are not intended to limit the scope of the present invention. Various objects and advantages of the present invention will become apparent to those skilled in the art from the following detailed description of the drawings and preferred embodiments.

### Brief Description of the Drawings

FIG. 1 shows the schematic diagram of an exemplary capture chip of the present application. Wherein, the capture probe (comprising a capture region, also referred to as capture domain) and the positioning probe (comprising spatial information region, also referred as spatial barcode) on the chip are located on two independent molecules.
FIG. 2 shows an exemplary implementation process of spatial transcriptomics of the present application.
FIG. 3 shows the detection results of chip probe.
FIG. 4 shows the detection results of cDNA using Bioanalyzer 2100.
FIG. 5 shows the spatial expression map of a mouse brain section.
FIG. 6 shows the cell types of a mouse brain section.
FIG. 7 shows the gene coverage analysis results of the captured transcriptome.

### Sequence Information

The sequences involved in the present application are described in the following table.

**Table 1: Sequence information**

| SEQ ID NO: | Description of sequence |
|---|---|
| 1 | Oligo sequence for blocking chip |
| | GTCTTAGGAAGACAA |
| 2 | Capture probe sequence |
| | CCTCCGACTGTGTGACTTAGACTCCTGCCACCTCCTGATGTG CTTTTTTTTTTTTTTTTTTTTTTTV |
| 3 | Cy5 probe sequence |
| | AAAAAAAAAAAAAAAAGCACA |
| 4 | TSO |
| | TTGTCTTCCTAAGACCGCTTGGNNNNN/rG//rG/iXNA_G |
| 5 | cDNA primer2 |
| | CCTCCGACTGTGTGACTTAGACTC |

| | |
|---|---|
| Note: V=A, C, or G; N=A, T, C, or G; "r" indicates that the nucleotide adjacent to its 3' position is a ribonucleotide; "iXNA" indicates a locked nucleic acid (LNA) nucleotide. | |

### Specific Models for Carrying Out the present invention

The present invention will now be described with reference to the following examples, which are intended to illustrate the present invention (but not limit the present invention), and are not intended to limit the scope of protection claimed by the present invention.

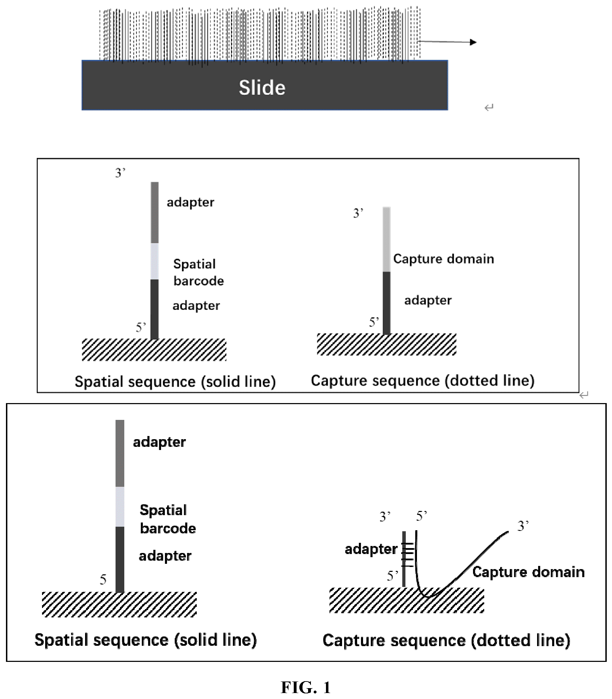

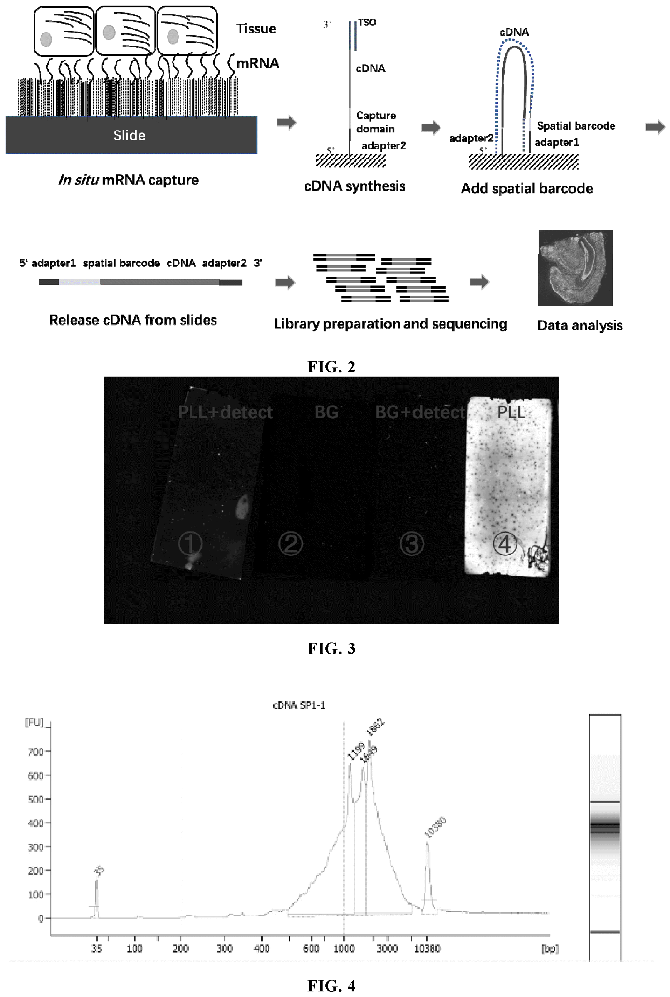

An exemplary implementation process for obtaining spatial transcriptomics information of samples based on the chip provided in the present application is shown in FIG. 2:
(1) preparation of the capture chip as shown in FIG. 1;
(2) capture of mRNA: Tissue sections are attached to the capture chip, and the tissue is processed to release mRNA molecules;
(3) The released mRNA molecules are captured by the capture probes on the chip, and cDNA molecules are synthesized in situ under the action of reverse transcriptase with terminal transferase activity; since the reverse transcriptase with terminal transferase activity is able to add a few extra cytosine nucleotides to the 3' end of a newly synthesized cDNA molecule, which form a complementary base pair with the 3' end of the TSO (template-switching oligonucleotide), the reverse transcriptase then "switches" template, using TSO as a template to continue synthesizing the cDNA molecule until reaching the 5' end of the TSO;
(4) the 3' end of the cDNA molecule can be captured by a positioning probe comprising location information in proximity thereto, and further extended, thereby adding spatial information to the cDNA molecule and synthesizing a cDNA molecule with spatial information;
(5) the cDNA molecule is collected from the chip and subjected to cDNA amplification, or PCR is performed directly on the chip to amplify the cDNA;
(6) library construction and sequencing;
(7) data analysis, in which the spatial information and RNA expression patterns are restored to their chip locations.

Furthermore, the applicant of the present application wishes to emphasize that, based on different designs of the capture region sequences, they can be designed to target a variety of target sequences, not limited to mRNA. Therefore, the chip provided in the present application can be used not only to obtain spatial transcriptomics information but also to acquire genomic, epigenomic, and multi-omics information, as well as information on specific target molecules.

### 1. Preparation of capture chip

An exemplary capture chip of the present application comprises a positioning probe and a capture probe. The capture probe comprises an adapter sequence and a capture sequence sequentially from 5' to 3', and the positioning probe comprises a first adapter sequence, a spatial positioning sequence (i.e., a positioning sequence), and a second adapter sequence sequentially from 5' to 3'.

In this example, an exemplary preparation method of the capture chip is illustrated, which comprises:
(a) providing a traditional chip commonly used in this field, which comprises both a positioning sequence and a capture sequence on the same probe (e.g., the traditional chip can be provided by purchase or self-preparation); the probe of the traditional chip comprises, in the direction from 5' to 3', a first adapter sequence, a spatial positioning sequence (i.e., a positioning sequence), a second adapter sequence, and a capture sequence;
(b) blocking the second adapter, for example, by hybridizing an oligonucleotide molecule capable of annealing to the second adapter sequence with the probe, thereby forming a double-stranded structure at the position of the second adapter of the probe;
(c) incubating an exonuclease with the product from (b), wherein the exonuclease possesses 3'→5' single-strand exonuclease activity; as a result, the capture sequence of the probe on the traditional chip is cleaved off by the exonuclease, forming a positioning probe that does not comprise the capture sequence but comprises the positioning sequence;
(d) on the chip obtained in (c) comprising the positioning probe, the capture probe is attached through a click chemistry reaction, thereby obtaining an exemplary capture chip of the present application that comprises both the capture probe and the positioning probe.

Specifically, the capture chip was prepared by the following steps:
(1) The conventional chip capture region was blocked and cleaved off to form a positioning probe. An oligo sequence, GTCTTAGGAAGACAA (SEQ ID NO: 1), was synthesized from Sangon Biotech. This oligo was prepared into a 1 µM oligo solution using 5× SSC (saline sodium citrate). A chip from the stereo-seq transcriptomics T kit (MGI, Cat. No. 111KT114) was taken out (on this conventional chip, the structure of each nucleic acid molecule from 5' to 3' is: adaptor 1 - spatial barcode sequence - adaptor 2 - capture sequence of poly T). Then, 100 µL of the 1 µM oligo solution was taken and added onto the chip and hybridized at room temperature for 30 minutes, thereby blocking the adaptor 2 on the nucleic acid molecules of the conventional chip. The hybridization solution was discarded by pipetting, and the chip was washed with 0.1× SSC. Subsequently, 50 µL of Exonuclease I (ThermoFisher: EN0581) reaction mix (5 µL Exonuclease I, 5 µL 10× Reaction buffer, 40 µL ddH₂O) was added and incubated at 37°C for 20 minutes, thereby cleaving off the capture sequence of poly T from the nucleic acid molecules on the conventional chip. The reaction mix was discarded, and the chip was thoroughly washed with 0.1× SSC. As a result, only "adaptor 1 - spatial barcode sequence - adaptor 2" remained on the chip.
(2) Modification of chip surface: 0.1% poly-L-lysine solution (PLL, Sigma P8920) was prepared and added onto the previously treated chip. The chip was incubated at 30°C for 3 hours to allow amino group modification on the surface of the chip. The reaction solution was then discarded, and the chip was washed with ddH₂O, followed by drying for 1 hour. NHS-PEG-N₃ reaction solution (Sigma JKA5088) was prepared according to the manufacturer's instructions and added onto the chip. The chip was incubated at 37°C for 5 hours to modify the surface of the chip with azido groups.
(3) Modification of chip capture probe oligo dT: A capture probe, 5'-CCTCCGACTGTGTGACTTAGACTCCTGCCACCTCCTGATGTGCTTTTTTTTTTTTTT TTTTTTTTV-3' (SEQ ID NO: 2), which comprised an adapter and oligo dT, was synthesized by Sangon. The 5' end of the probe was modified with DBCO (dibenzocyclooctyne). The probe was diluted to 1 µM with PBS, added onto the chip, and reacted overnight at 37°C. This allowed the azido groups on the surface of the chip to undergo a click reaction with the DBCO-modified probe, thereby linking the oligo dT probe onto the chip and obtaining a chip comprising the capture probe.
(4) Detection of chip probe: A detection probe, AAAAAAAAAAAAAAAAGCACA (SEQ ID NO: 3), which was modified with a cy5 fluorescent tag at the 3' end, was used for hybridization and imaging, thereby assessing the synthesis of capture probes on the chip and the removal of the capture regions on the chip from step 1. The results are shown in FIG. 3: Panel ① showed the picture of the PLL-modified chip obtained from step (2) after the addition of the detection probe; Panel ② showed the background of the positioning probe chip obtained from step (1) (without the addition of the detection probe); Panel ③ showed the picture of the positioning probe chip obtained from step (1) after the addition of the detection probe; Panel ④ showed the fluorescence signal on the chip linked with oligo dT obtained from step (3) following the addition of the detection probe. The results indicated that Panels ② and ③ demonstrated successful removal of the original poly T capture regions on the commercial chip, while Panel ④ confirmed that the newly synthesized oligo dT capture probes were successfully modified onto the chip.

### 2. Capture of mRNA molecule

Referring to the instructions of the stereo-seq transcriptomics T kit, tissue sections, such as mouse brain sections, were attached to the chip. After the sections were subjected to permeabilization and other treatments, mRNA was released, allowing the mRNA in the tissue to be captured by the capture probes on the chip.

### 3. In situ cDNA synthesis

Following the instructions of the stereo-seq transcriptomics T kit, the following reverse transcription reaction system was prepared, and reacted at 42°C for 5 hours.

| Reagent | 1× (µL) |
|---|---|
| Reverse Reagent (1000028507) | 80 |
| RT Additive(1000028502) | 5 |
| RT Oligo (TSO), SEQ ID NO: 4: TTGTCTTCCTAAGACCGCTTGGNNNNN/rG//rG//iXNA_G/ | 5 |
| RI(1000028499) | 5 |
| Reverse T enzyme (1000028509) | 5 |
| Total | 100 |

### 4. Addition of spatial information

After cDNA synthesis was completed, the reaction solution was discarded. Then, TR buffer (1000028507) was added according to the manufacturer's instructions and incubated for 30 minutes to remove the tissue. A formamide solution was prepared, added onto the chip surface, and incubated at 80°C for 3 hours. The reaction solution was then removed by pipetting to remove mRNA, and the chip surface was washed with ddH₂O. 5× SSC reaction solution was added onto the chip surface and incubated at 37°C for 30 minutes. After removing the reaction solution, the chip surface was washed with 0.1× SSC. The following reaction solution was then added and incubated at 37°C for 3 hours for extension to obtain spatial information.

| Reagent | 1x (µL) |
|---|---|
| Reverse Reagent (1000028507) | 90 |
| RT Additive(1000028502) | 5 |
| Reverse T enzyme (1000028509) | 5 |
| Total | 100 |

### 5. cDNA release and cDNA amplification

A 100 µL solution of 100 mM KOH was prepared and added to the chip. The reaction was carried out at 55 °C for 3 hours. The reaction supernatant was collected into a PCR tube, and the chip was washed with 50 µL of ddH₂O. The washing solution was combined with the supernatant in the PCR tube. The reaction mixture was neutralized with 0.1 M HCl to pH 8.5. After mixing well, the solution was divided into three tubes for PCR reactions using the reaction mixture described below. PCR was performed according to the manufacturer's instructions. The obtained cDNA was analyzed using a Bioanalyzer 2100, and the results are shown in FIG. 4.

| Reagent | 1x (µL) |
|---|---|
| The neutralized product as described above | 50 |
| cDNA primer (1000028513) | 2.5 |
| cDNA primer2: CCTCCGACTGTGTGACTTAGACTC (SEQ ID NO: 5) | 2.5 |
| cDNA amplification Mix (1000028514) | 45 |
| Total | 100 |

### 6. Library construction and sequencing

Following the instructions of the stereo-seq transcriptomics T kit, fragmentation of cDNA library was carried out to prepare DNB.

Following the instructions of MGISEQ 2000 sequencer, the DNB was loaded onto the sequencing chip of the MGISEQ 2000, and sequencing was performed. A 25 bp read was set for decoding the spatial barcode, followed by a 22 bp dark reaction. Then, a 5 bp sequencing reaction was carried out to obtain the molecular tag (MID) sequence. Subsequently, a 50 bp to 100 bp sequencing reaction was performed to acquire the information of cDNA 5' end transcript.

### 7. Data analysis

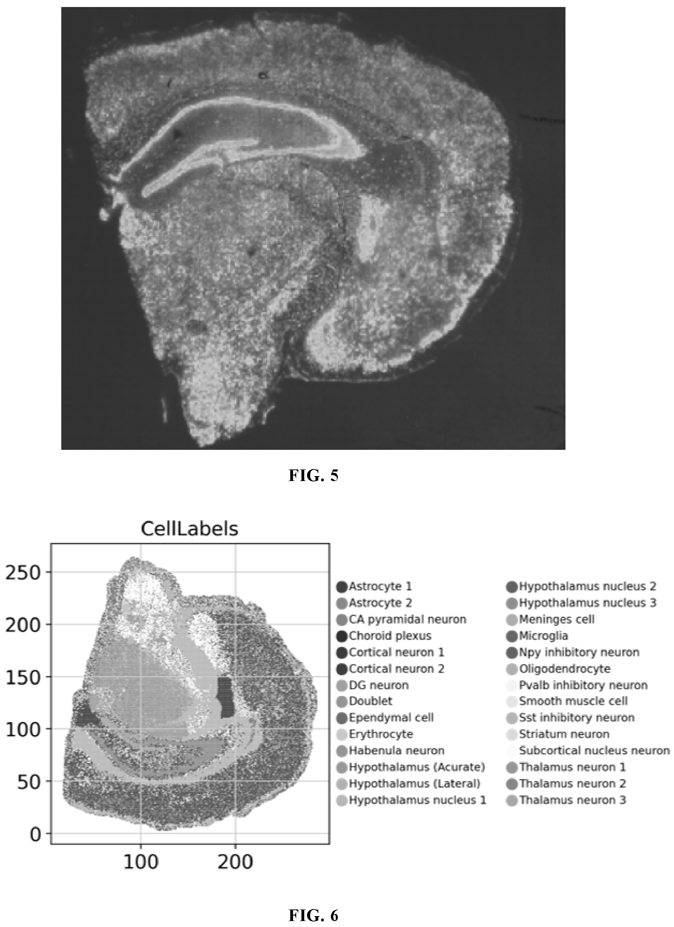

(1) The website http://stereomap.cngb.org/Stereo-Draftsman/report/index was logged into, and data analysis was performed by following the operating guidelines provided on the website. The first 25 bp of the read1 sequence (obtained from one-strand sequencing) from step 6 were aligned with the 25 bp location information of the capture chip from step 1. The reads that could be successfully aligned to the location information on the chip were retained and then mapped to their corresponding chip positions. The 46 bp starting sequences corresponding to the chip-mapped reads were identified as the cDNA sequences. These sequences were then reverse-complemented and aligned to the mouse brain genome. Based on the MID information obtained from 41 bp to 45 bp, duplicate reads were removed, and the expression count of each gene in the mouse brain was finally obtained.
(2) Using the expression counts of each gene, further mapping was performed, and a spatial expression atlas of the mouse brain section was obtained as shown in FIG. 5. Spatial clustering analysis was conducted on the expression levels, and the cell types of the mouse brain section were obtained as shown in FIG. 6.
(3) Capture and analysis of 5' end transcripts

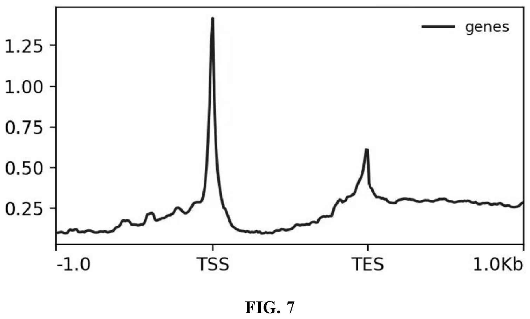

A gene coverage analysis was performed on the captured transcriptomes. Most of the captured transcriptomes were found to be concentrated in the transcription start site (TSS) region, demonstrating that this protocol enabled the capture of 5' end transcripts, as shown in FIG. 7.

Although specific embodiments of the present invention have been described in detail, those skilled in the art will understand that various modifications and variations can be made to the details based on all the disclosed teachings, and all such changes are within the scope of protection of the present invention. The entire scope of the present invention is given by the appended claims and any equivalents thereof.

## Claims

1. A nucleic acid array for detecting spatial information of a nucleic acid in a sample, which comprises a solid support, wherein one or more types of capture probes and at least two types of positioning probes are attached to the solid support;
the capture probes and the positioning probes are each independently attached to the solid support;
each type of the positioning probe occupies a different position on the solid support;
the positioning probe comprises a first universal sequence and a positioning sequence, wherein the positioning sequence has a nucleotide sequence corresponding to the position of the type of positioning probe on the solid support;
the capture probe comprises a capture sequence, wherein the capture sequence is capable of annealing to the nucleic acid molecule to be captured and initiating an extension reaction.

2. The nucleic acid array according to claim 1, wherein the first universal sequence is located 3' of the positioning sequence;
preferably, the positioning probe further comprises a second universal sequence;
preferably, the second universal sequence is located 5' of the positioning sequence;
preferably, the second universal sequences comprised in different types of positioning probes attached to the solid support are the same or different; preferably, the second universal sequences comprised in different types of positioning probes attached to the solid support are the same;
preferably, the positioning probe does not comprise or further comprises a molecular identifier (MID);
preferably, the positioning probe further comprises an MID sequence;
preferably, the MID sequence is located 5' of the first universal sequence, and/or, the MID sequence is located 3' of the second universal sequence;
preferably, the MID sequences comprised in the same type of positioning probes are different from each other.

3. The nucleic acid array according to claim 1 or 2, wherein the capture sequence is located at the 3' end of the capture probe;
preferably, the capture sequence has a free hydroxyl (-OH) at the 3' end;
preferably, the capture probe further comprises a consensus sequence;
preferably, the consensus sequences comprised in different capture probes attached to the solid support are the same or different; preferably, the consensus sequences comprised in different capture probes attached to the solid support are the same;
preferably, the consensus sequence is located 5' of the capture sequence.

4. The nucleic acid array according to any one of claims 1 to 3, wherein the capture probe comprises a single-stranded region that comprises the capture sequence;
preferably, the capture probe is present in a single-stranded or duplex form that comprises the single-stranded region;
preferably, the capture probe is present in a single-stranded form, and the capture sequence is located at the 3' end of the capture probe; or,
the capture probe is present in a duplex form that comprises a single-stranded region, comprising a first strand directly attached to the solid support, and a second strand hybridized to the first strand; and, the 3' end of the second strand comprises a single-stranded region, and the capture sequence is located at the 3' end of the single-stranded region.

5. The nucleic acid array according to any one of claims 1 to 4, comprising one or more of features selected from the following:
(1) the positioning sequence is a nucleotide sequence consisting of 5 to 50 (e.g., 5 to 25, 5 to 35, 5 to 45, 10 to 25, 10 to 35, 10 to 45, 15 to 25, 15 to 35, 15 to 45, 20 to 25, 20 to 35, or 20 to 45) random nucleotides; preferably, each random nucleotide is independently any one of deoxyribonucleotides A, C, G, and T;
(2) the solid support is selected from the group consisting of latex bead, dextran bead, polystyrene surface, polypropylene surface, polyacrylamide gel, gold surface, glass surface, chip, sensor, electrode, and silicon wafer; preferably, the solid support is a chip; preferably, the solid support is capable of being used in a sequencing platform; preferably, the solid support is a sequencing chip, such as a sequencing chip for use in an Illumina, MGI, or Thermo Fisher sequencing platform;
(3) the positioning probes of the same type occupy the same region on the surface of the solid support, the positioning probes of different types occupy different regions on the surface of the solid support, and the center-to-center distance between any two adjacent regions is less than 1 µm (e.g., less than 900 nm, less than 800 nm, less than 700 nm, less than 600 nm, less than 500 nm, less than 400 nm, less than 300 nm, less than 200 nm);
(4) the first universal sequence is capable of annealing to (i) the nucleic acid molecule captured by the capture sequence, or (ii) a nucleic acid molecule derived from (i);
(5) the first universal sequences comprised in different types of positioning probes attached to the solid support are the same or different;
(6) the solid support is capable of releasing the positioning probe and/or capture probe spontaneously or upon exposure to one or more stimuli (e.g., temperature change, pH change, exposure to a specific chemical or phase, exposure to light, a reducing agent, etc.).

6. The nucleic acid array according to any one of claims 1 to 5, wherein the positioning probe and/or the capture probe are covalently or non-covalently attached to the solid support;
preferably, the positioning probe and/or the capture probe are covalently attached to the solid support;
preferably, the positioning probe and the capture probe are each independently attached to the solid support through the same or different click chemistry reactions;
preferably, the molecule or group pair capable of undergoing the click chemistry reaction is selected from the group consisting of: alkynyl/azido, azido/cyano, amine/ene, thiol/ene, thiol/alkynyl, aldehyde/1,3-diol, ketone/1,3-diol; preferably, the molecule or group pair capable of undergoing the click chemistry reaction is azido/alkynyl;
for example, the solid support is surface-modified with a molecule or group X, and the positioning probe is modified with a molecule or group Y, wherein the molecule or group X is capable of undergoing a click chemistry reaction with the molecule or group Y, and the positioning probe is attached to the solid support via the click chemistry reaction between the molecule or group X and the molecule or group Y; and/or, the solid support is surface-modified with the molecule or group X', and the capture probe is modified with a molecule or group Y', wherein the molecule or group X' is capable of undergoing a click chemistry reaction with the molecule or group Y', and the capture probe is attached to the solid support via the click chemistry reaction between the molecule or group X' and the molecule or group Y';
preferably, the molecule or group pairs X/Y and X'/Y' are each independently selected from the group consisting of: alkynyl/azido, azido/cyano, amine/ene, thiol/ene, thiol/alkynyl, aldehyde/1,3-diol, ketone/1,3-diol, azido/alkynyl, cyano/azido, ene/amine, ene/thiol, alkynyl/thiol, 1,3-diol/aldehyde, 1,3-diol/ketone;
preferably, the solid support is surface-modified with azido, the positioning probe and/or the capture probe are modified with 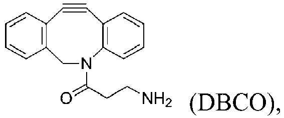 and the positioning probe and/or the capture probe are attached to the solid support via a click chemistry reaction between the azido and the DBCO.

7. The nucleic acid array according to any one of claims 1 to 6, wherein at least 100 of the capture probes, preferably 500 to 10000 of the capture probes, are distributed in proximity to each type of the positioning probes;
preferably 500 to 10000 of the capture probes, are distributed around the center of the location occupied by each type of the positioning probes on the solid support (e.g., the center of the area occupied by each type of the positioning probes on the surface of the solid support) within a radius of 200 nm to 300 nm.

8. The nucleic acid array according to any one of claims 1 to 7, wherein the nucleic acid array comprises one or more types of the capture probes;
preferably, different types of capture probes comprise different capture sequences;
preferably, the nucleic acid molecule to be captured is an RNA (e.g., an mRNA), and the capture sequence of the capture probe comprises a poly(dT) sequence or a random oligonucleotide sequence; or,
the nucleic acid molecule to be captured is a target nucleic acid (e.g., a target DNA and/or RNA) or a nucleic acid molecule derived from the target nucleic acid, the target nucleic acid or the nucleic acid molecule derived from the target nucleic acid has a target nucleotide sequence comprised in the target nucleic acid, and the capture sequence of the capture probe comprises a sequence capable of specifically annealing to the target nucleotide sequence; or,
the nucleic acid molecule to be captured is an RNA (e.g., an mRNA) and a target nucleic acid (e.g., a target DNA and/or RNA), the nucleic acid array comprises a first capture probe capable of capturing RNA (e.g., mRNA) and a second capture probe capable of capturing the target nucleic acid or a nucleic acid molecule derived from the target nucleic acid, and the target nucleic acid or the nucleic acid molecule derived from the target nucleic acid has a target nucleotide sequence comprised in the target nucleic acid; wherein the capture sequence of the first capture probe comprises a poly(dT) sequence or a random oligonucleotide sequence, and the capture sequence of the second capture probe comprises a sequence capable of specifically annealing to the target nucleotide sequence.

9. A method for preparing the nucleic acid array according to any one of claims 1 to 8, which comprises the following:
(A) attaching and/or synthesizing a positioning probe on a solid support, the positioning probe being as defined in claim 1, 2, or 5; and,
(B) attaching and/or synthesizing a capture probe on a solid support, the capture probe being as defined in any one of claims 1, 3 to 5, or 8;
wherein, the (A) and the (B) may be performed in any order or simultaneously (e.g., simultaneously in the same reaction system);
for example, the (A) is performed before the (B), and the solid support in the (B) is a solid support with the positioning probe attached thereto;
for example, the (B) is performed before the (A), and the solid support in the step (A) is a solid support with the capture probe attached thereto;
for example, the (A) and the (B) are performed simultaneously, and the solid support in the (A) and the (B) is the same solid support.

10. The method according to claim 9, wherein the (A) comprises:
(1) providing: (a) the positioning probe in free form, wherein the positioning probe is modified with a molecule or group A; and (b) the solid support, wherein the solid support is surface-modified with a molecule or group B, wherein the molecule or group A is capable of forming a linkage (e.g., covalent or non-covalent linkage) with the molecule or group B; and,
(2) contacting the positioning probe with the solid support under a condition suitable for forming a linkage between the molecule or group A and the molecule or group B, thereby obtaining a solid support with the positioning probe attached thereto;
preferably, the molecule or group A is capable of undergoing a click chemistry reaction with the molecule or group B;
preferably, the molecule or group pair A/B is selected from the group consisting of: alkynyl/azido, azido/cyano, amine/ene, thiol/ene, thiol/alkynyl, aldehyde/1,3-diol, ketone/1,3-diol, azido/alkynyl, cyano/azido, ene/amine, ene/thiol, alkynyl/thiol, 1,3-diol/aldehyde, 1,3-diol/ketone;
preferably, the molecule or group pair A/B is azido/alkynyl or alkynyl/azido;
preferably, the positioning probe is modified with 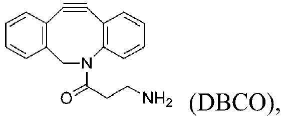 the solid support is surface-modified with azido, and the positioning probe is attached to the solid support via a click chemistry reaction between DBCO and azido.

11. The method according to claim 9, wherein the (A) comprises:
(1) providing at least two types of carriers, each type of carrier comprising at least one copy of a carrier sequence, wherein the carrier sequence comprises: a complementary sequence to a positioning sequence and a complementary sequence to a first universal sequence; the first universal sequence and the positioning sequence are as defined in any one of claims 1, 2, and 5; wherein the complementary sequences to the positioning sequences of each type of the carrier sequences are different from each other;
(2) placing at least two types of the carriers onto the surface of the solid support;
(3) providing a immobilization primer, and using the carrier sequence as a template to carry out a nucleic acid polymerization reaction to generate an extension product, wherein the extension product is the positioning probe; wherein the immobilization primer is capable of annealing to the carrier sequence and initiating an extension reaction; and
(4) attaching the immobilization primer to the surface of the solid support;
wherein steps (3) and (4) are performed in any order (for example, step (3) is performed before or after step (4), or step (3) and step (4) are performed simultaneously);
preferably, the carrier sequence comprises, in the direction from 5' to 3', the complementary sequence to the universal sequence, and the complementary sequence to the positioning sequence;
preferably, the extension product comprises, in the direction from 5' to 3', the positioning sequence, and the first universal sequence;
preferably, each type of the carriers is a DNB formed by a concatemer of multiple copies of the carrier sequence;
preferably, the method optionally comprises step (5): digesting the carrier sequence, and/or separating the extension product in step (3) from the carrier sequence annealed thereto;
preferably, the carrier is provided in step (1) by the following steps:
(i) providing a template of the carrier sequence, wherein the template comprises a complementary sequence to the carrier sequence;
(ii) using the template as a template, performing a nucleic acid amplification reaction to obtain an amplification product of the template, wherein the amplification product comprises at least one copy of the carrier sequence; preferably, rolling circle replication is performed to obtain a DNB formed by a concatemer of the carrier sequence.

12. The method according to claim 11, wherein the carrier sequence further comprises a complementary sequence to a second universal sequence or portion sequence thereof (e.g., the carrier sequence further comprises a complementary sequence to the second universal sequence or 3' end portion sequence thereof), the second universal sequence is as defined in claim 2;
preferably, the complementary sequence to the second universal sequence or portion sequence thereof is located 3' of the complementary sequence to the positioning sequence;
preferably, the immobilization primer comprises the second universal sequence or portion sequence thereof (e.g., the immobilization primer comprises the second universal sequence or 5' end portion sequence thereof);
preferably, the extension product comprises, in the direction from 5' to 3', the second universal sequence, the positioning sequence, and the first universal sequence, sequentially;
preferably, the carrier sequence does not comprise or further comprises a template sequence of an MID sequence;
preferably, the carrier sequence further comprises a template sequence for an MID sequence;
preferably, the template sequence for the MID sequence is located 3' of the complementary sequence to the first universal sequence, and/or the template sequence for the MID sequence is located 5' of the complementary sequence to the second universal sequence or portion sequence thereof; preferably, the MID sequence consists of 5 to 50 (e.g., 5 to 25, 5 to 35, 5 to 45, 10 to 25, 10 to 35, 10 to 45, 15 to 25, 15 to 35, 15 to 45, 20 to 25, 20 to 35, or 20 to 45) degenerate deoxyribonucleotide residues.

13. The method according to claim 11 or 12, wherein the immobilization primer is covalently or non-covalently attached to the solid support;
preferably, the immobilization primer is covalently attached to the solid support;
preferably, the immobilization primer is attached to the solid support via a click chemistry reaction;
preferably, the molecule or group pair capable of undergoing the click chemistry reaction is selected from the group consisting of: alkynyl/azido, azido/cyano, amine/ene, thiol/ene, thiol/alkynyl, aldehyde/1,3-diol, ketone/1,3-diol; preferably, the molecule or group pair capable of undergoing the click chemistry reaction is azido/alkynyl;
preferably, the immobilization primer is modified with 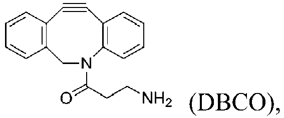 the solid support is surface-modified with azido, and the immobilization primer is attached to the solid support via a click chemistry reaction between DBCO and azido.

14. The method according to any one of claims 9 to 13, wherein the capture probe is present in a single-stranded form comprising a single-stranded region that comprises a capture sequence, wherein the capture sequence is as defined in any one of claims 1, 3 to 5, 8, and the (B) comprises:
(1) providing: (a) the capture probe in free form, wherein the capture probe is modified with a molecule or group A'; and (b) the solid support, wherein the solid support is surface-modified with a molecule or group B', and the molecule or group A' is capable of forming a linkage (e.g., a covalent or non-covalent linkage) with the molecule or group B'; and
(2) contacting the capture probe with the solid support under a condition suitable for forming the linkage between the molecule or group A' and the molecule or group B', thereby obtaining a solid support with the capture probe attached thereto;
preferably, the capture sequence is located at the 3' end of the capture probe.

15. The method according to any one of claims 9 to 13, wherein the capture probe is present in a duplex form comprising a single-stranded region that comprises a capture sequence, wherein the capture sequence is as defined in any one of claims 1, 3 to 5, 8, and the (B) comprises:
(I)
(1) providing: (a) a free first strand of the capture probe, wherein the first strand is modified with a molecule or group A'; and (b) the solid support, wherein the solid support is surface-modified with a molecule or group B', and the molecule or group A' is capable of forming a linkage (e.g., covalent and/or non-covalent linkage) with the molecule or group B'; and (c) a free second strand of the capture probe, wherein the second strand comprises the capture sequence and is capable of annealing to the first strand to form a duplex;
(2) contacting the first strand with the solid support under a condition suitable for forming a linkage between the molecule or group A' and the molecule or group B', thereby obtaining a solid support having the first strand attached thereto; and
(3) contacting the free second strand with the solid support having the first strand attached thereto from step (2) under a condition allowing annealing, thereby obtaining a solid support with the capture probe attached thereto;
or,
(II)
(1) providing: (a) a duplex formed by annealing a first strand and a second strand of the capture probe, wherein the first strand is modified with a molecule or group A', and the second strand comprises the capture sequence; and (b) a solid support, wherein the solid support is surface-modified with a molecule or group B', the molecule or group A' is capable of forming a linkage (e.g., covalent and/or non-covalent linkage) with the molecule or group B'; and
(2) contacting the duplex with the solid support under a condition suitable for forming a linkage between the molecule or group A' and the molecule or group B', thereby obtaining a solid support with the capture probe attached thereto;
preferably, in the duplex formed by annealing the first strand to the second strand of the capture probe, the second strand comprises the capture sequence at its 3' end, and the capture sequence is not annealed to the first strand.

16. The method according to claim 14 or 15, wherein the molecule or group A' is capable of undergoing a click chemistry reaction with the molecule or group B';
preferably, the molecule or group pair A'/B' is selected from the group consisting of: alkynyl/azido, azido/cyano, amine/ene, thiol/ene, thiol/alkynyl, aldehyde/1,3-diol, ketone/1,3-diol, azido/alkynyl, cyano/azido, ene/amine, ene/thiol, alkynyl/thiol, 1,3-diol/aldehyde, 1,3-diol/ketone;
preferably, the molecule or group pair A'/B' is azido/alkynyl or alkynyl/azido;
preferably, the first strand of the capture probe is modified with 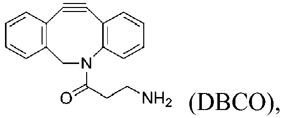 the solid support is surface-modified with azido, and the first strand of the capture probe is attached to the solid support by a click chemistry reaction between DBCO and azido.

17. A method for preparing the nucleic acid array according to any one of claims 1 to 8, which comprises the following:
(A) providing a nucleic acid array to be processed, wherein the nucleic acid array to be processed comprises a solid support, the solid support is attached with at least two types of oligonucleotide molecules;
each type of oligonucleotide molecules occupies a different position on the solid support;
the oligonucleotide molecule comprises, in the direction from 5' to 3', a positioning sequence and a first universal sequence sequentially, wherein the positioning sequence has a nucleotide sequence corresponding to the position of the type of the oligonucleotide molecule on the solid support; the first universal sequence is as defined in any one of claims 1, 2, and 5;
and,
(B) attaching and/or synthesizing a capture probe on the solid support comprised in the nucleic acid array to be processed, wherein the capture probe is as defined in any one of claims 1, 3 to 5, and 8.

18. The method according to claim 17, wherein the oligonucleotide molecule does not comprise a capture sequence located 3' of the first universal sequence, the capture sequence being as defined in any one of claims 1, 3 to 5, and 8.

19. The method according to claim 17, wherein the oligonucleotide molecule comprises a capture sequence located 3' of the first universal sequence, the capture sequence being as defined in any one of claims 1, 3 to 5, and 8, and the method further comprises (B'): removing the capture sequence comprised in the oligonucleotide molecule;
wherein, the (B) and the (B') can be performed in any order or simultaneously (e.g., simultaneously in the same reaction system).

20. The method according to claim 19, wherein in the (B'), removing the capture sequence comprised in the oligonucleotide molecule comprises the following steps:
(i) providing a blocking probe, wherein the blocking probe is capable of annealing to (a) the first universal sequence of the oligonucleotide molecule or a portion sequence thereof (e.g., a 3' end portion sequence of the first universal sequence), or (b) a sequence in the oligonucleotide molecule, which is located 5'of the capture sequence and 3' of the first universal sequence, or (c) a combination of (a) and (b);
(ii) contacting the blocking probe with the nucleic acid array to be processed that comprises the oligonucleotide molecule under a condition suitable for annealing the blocking probe to the oligonucleotide molecule;
(iii) contacting an exonuclease with the product of step (ii) under a condition that allows the exonuclease to exert its cleavage activity; wherein the exonuclease has single-stranded nucleic acid-specific 3'-to-5' exonucleolytic activity;
preferably, the exonuclease is exonuclease I;
preferably, the (B') further comprises a step of removing the blocking probe (e.g., by melting the blocking probe away from the oligonucleotide molecule, thereby removing the blocking probe);
preferably, in the method, the (B) is performed after the (B').

21. The method according to any one of claims 17 to 20, wherein the oligonucleotide molecule further comprises a second universal sequence, the second universal sequence being as defined in claim 2 or 5;
preferably, the second universal sequence is located 5' of the positioning sequence;
preferably, the oligonucleotide molecule does not comprise or further comprises an MID sequence;
preferably, the oligonucleotide molecule further comprises an MID sequence; preferably, the MID sequence is located 5' of the first universal sequence, and/or, the MID sequence is located 3' of the second universal sequence; preferably, the MID sequences comprised in the same type of oligonucleotide molecules are different from each other.

22. The method according to any one of claims 17 to 21, wherein, in the nucleic acid array to be processed, the oligonucleotide molecules are covalently or non-covalently attached to the solid support;
preferably, the oligonucleotide molecules are covalently attached to the solid support;
preferably, the oligonucleotide molecules are attached to the solid support via a click chemistry reaction;
preferably, the molecule or group pair capable of undergoing the click chemistry reaction is selected from the group consisting of: alkynyl/azido, azido/cyano, amine/ene, thiol/ene, thiol/alkynyl, aldehyde/1,3-diol, ketone/1,3-diol; preferably, the molecule or group pair capable of undergoing the click chemistry reaction is azido/alkynyl;
preferably, the solid support is surface-modified with azido, the oligonucleotide molecules are modified with 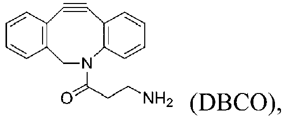 and the oligonucleotide molecules are attached to the solid support via a click chemistry reaction between the azido and the DBCO.

23. The method according to any one of claims 17 to 22, wherein the capture probe is present in a single-stranded form comprising a single-stranded region that comprises a capture sequence, wherein the capture sequence is as defined in any one of claims 1, 3 to 5, 8, and the (B) comprises:
(1) providing: (a) the capture probe in the free form, wherein the capture probe is modified with a molecule or group A'; and (b) the nucleic acid array to be processed or the nucleic acid array processed in the (B'), wherein the solid support of the nucleic acid array is surface-modified with a molecule or group B', and the molecule or group A' is capable of forming a linkage (e.g., a covalent or non-covalent linkage) with the molecule or group B'; and,
(2) contacting the capture probe with the solid support under a condition suitable for forming a linkage between the molecule or group A' and the molecule or group B', thereby obtaining a solid support with the capture probe attached thereto;
preferably, the capture sequence is located at the 3' end of the capture probe.

24. The method according to any one of claims 17 to 23, wherein the capture probe is in the form of a duplex form comprising a single-stranded region that comprises a capture sequence, wherein the capture sequence is as defined in any one of claims 1, 3 to 5, 8, and the (B) comprises:
(I)
(1) providing: (a) a free first strand of the capture probe, wherein the first strand is modified with a molecule or group A'; and (b) the nucleic acid array to be processed or the nucleic acid array processed in the (B'), wherein the solid support of the nucleic acid array is surface-modified with a molecule or group B', and the molecule or group A' is capable of forming a linkage (e.g., covalent and/or non-covalent linkage) with the molecule or group B'; and (c) a free second strand of the capture probe, wherein the second strand comprises the capture sequence and is capable of annealing to the first strand to form a duplex;
(2) contacting the first strand with the solid support under a condition suitable for forming a linkage between the molecule or group A' and the molecule or group B', thereby obtaining a solid support having the first strand attached thereto; and
(3) contacting the free second strand with the solid support having the first strand attached thereto obtained in step (2) under a condition that allows annealing, thereby obtaining a solid support with the capture probe attached thereto;
or,
(II)
(1) providing: (a) a duplex formed by annealing a first strand to a second strand of the capture probe, wherein the first strand is modified with a molecule or group A', and the second strand comprises the capture sequence; and (b) the nucleic acid array to be processed or the nucleic acid array processed in the (B'), wherein the solid support of the nucleic acid array is surface-modified with a molecule or group B', and the molecule or group A' is capable of forming a linkage (e.g., covalent and/or non-covalent linkage) with the molecule or group B'; and
(2) contacting the duplex with the solid support under a condition suitable for forming a linkage between the molecule or group A' and the molecule or group B', thereby obtaining a solid support with the capture probe attached thereto;
preferably, in the duplex formed by annealing the first strand to the second strand of the capture probe, the second strand comprises the capture sequence at its 3' end, and the capture sequence is not annealed to the first strand.

25. The method according to claim 23 or 24, wherein the molecule or group A' is capable of undergoing a click chemistry reaction with the molecule or group B';
preferably, the molecule or group pair A'/B' is selected from the group consisting of: alkynyl/azido, azido/cyano, amine/ene, thiol/ene, thiol/alkynyl, aldehyde/1,3-diol, ketone/1,3-diol, azido/alkynyl, cyano/azido, ene/amine, ene/thiol, alkynyl/thiol, 1,3-diol/aldehyde, 1,3-diol/ketone;
preferably, the molecule or group pair A'/B' is azido/alkynyl or alkynyl/azido;
preferably, the first strand of the capture probe is modified with 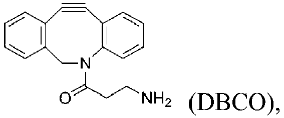 the solid support is surface-modified with azido, and the first strand of the capture probe is attached to the solid support by a click chemistry reaction between DBCO and azido.

26. A method for detecting spatial information of a nucleic acid in a sample, comprising the following steps:
(1) providing the nucleic acid array according to any one of claims 1 to 8;
(2) contacting the nucleic acid array with a sample to be tested under a condition allowing annealing, such that a nucleic acid derived from the sample to be tested anneals to a capture sequence in a capture probe of the nucleic acid array;
(3) performing a nucleic acid polymerization reaction under a condition allowing nucleic acid polymerization to generate a first extension product, wherein the first extension product comprises the capture sequence and a complementary sequence to a nucleic acid molecule or portion sequence thereof capable of annealing to the capture sequence, and the first extension product is attached via the capture sequence comprised therein to the solid support;
(4) annealing the first extension product attached to the solid support to a positioning probe in proximity thereto on the nucleic acid array under a condition allowing annealing;
wherein, a first universal sequence of the positioning probe is capable of annealing to the first extension product; and the first universal sequence is located 3' of the positioning sequence of the positioning probe;
(5) performing a nucleic acid polymerization reaction under a condition allowing nucleic acid polymerization to generate a second extension product and/or a complementary strand thereof, wherein the second extension product comprises: (a) the positioning sequence of the positioning probe and a complementary sequence to the first extension product or portion sequence thereof, or (b) the sequence of the first extension product or portion sequence thereof, and a complementary sequence to the positioning sequence of the positioning probe; the positioning sequence or complementary sequence thereof comprised in the second extension product serves as its spatial information tag, and the positioning sequence or complementary sequence thereof comprised in the complementary strand to the second extension product serves as its spatial information tag, so that the position of the nucleic acid derived from the sample to be tested in the sample to be tested is mapped to the position of the positioning probe corresponding to the positioning sequence; thereby obtaining a nucleic acid molecule comprising a spatial information tag; and
(6) analyzing the sequence of: (i) the nucleic acid molecule comprising the spatial information tag obtained in step (5), and/or (ii) a nucleic acid molecule derived from the nucleic acid molecule comprising the spatial information tag of (i);
preferably, the spatial information of the nucleic acid comprises the localization, distribution, and/or abundance of the nucleic acid.

27. The method according to claim 26, wherein, in step (3), the nucleic acid molecule annealed to the capture sequence is used as a template in the nucleic acid polymerization reaction to extend the capture sequence;
preferably, the capture sequence is located at the 3' end of the capture probe, and/or, the capture sequence has a free hydroxyl (-OH) at the 3' end.

28. The method according to claim 26 or 27, wherein, in step (5), the first extension product is used as a template in the polymerization reaction to extend the positioning probe; and/or the positioning probe is used as a template to extend the first extension product;
for example, in step (5), the first extension product is used as a template in the polymerization reaction to extend the positioning probe, so as to generate the second extension product and/or a complementary strand thereof, the second extension product comprising the positioning sequence of the positioning probe and a complementary sequence to the first extension product or portion sequence thereof; preferably, the first universal sequence of the positioning probe is located at the 3' end of the positioning probe, and/or, the first universal sequence of the positioning probe has a free hydroxyl (-OH) at the 3' end;
for example, in step (5), the positioning probe is used as a template in the polymerization reaction to extend the first extension product, so as to generate the second extension product and/or a complementary strand thereof, the second extension product comprising the sequence of the first extension product or portion sequence thereof and a complementary sequence to the positioning sequence of the positioning probe; preferably, the first universal sequence of the positioning probe is or is not located at the 3' end of the positioning probe, and/or, the 3' end of the first universal sequence of the positioning probe is blocked or unblocked;
for example, in step (5), during the polymerization reaction, the first extension product and the positioning probe are used as templates for each other to extend the first extension product and the positioning probe, so as to generate the second extension product and/or a complementary strand thereof, the second extension product comprising the positioning sequence of the positioning probe and a complementary sequence to the first extension product or portion sequence thereof, and the second extension product comprising the sequence of the first extension product or portion sequence thereof and a complementary sequence to the positioning sequence of the positioning probe; preferably, the first universal sequence of the positioning probe is located at the 3' end of the positioning probe, and/or the first universal sequence of the positioning probe has a free hydroxyl (-OH) at the 3' end.

29. The method according to any one of claims 26 to 28, wherein, in step (6), analysis is performed on the sequence of the nucleic acid molecule that is attached to the solid support of the nucleic acid array and comprises the spatial information tag.

30. The method according to any one of claims 26 to 28, wherein, before step (6) and after step (5), the method further comprises step pre-(6): releasing at least a part of the nucleic acid molecules comprising the spatial information tag from the surface of the nucleic acid array;
preferably, in step (6), analysis is performed on the sequence of (i) the nucleic acid molecule comprising the spatial information tag released in step pre-(6), and/or, (ii) a nucleic acid molecule derived from the nucleic acid molecule of (i) and comprising the spatial information tag;
preferably, in step pre-(6), the nucleic acid molecule is released from the surface of the solid support by the following method: (i) nucleic acid cleavage; and/or, (ii) denaturation;
preferably, after step pre-(6) and before step (6), the method further comprises a step of amplifying the released nucleic acid molecule;
preferably, before performing step (6), the method further comprises a step of purifying the released nucleic acid molecule.

31. The method according to any one of claims 26 to 30, comprising one or more features selected from the following:
(i) in step (1), the nucleic acid array is provided by the method according to any one of claims 9 to 25;
(ii) in step (2), the sample to be tested is processed (e.g., by permeabilization or cell lysis) to release a nucleic acid derived from the sample to be tested, and allow it to anneal to the capture sequence;
(iii) after step (3) and before step (4), the method further comprises a step of removing a template strand bound to the first extension product (e.g., removing a template strand bound to the first extension product by enzyme digestion or melting);
(iv) after step (3) and before step (4), the method further comprises a step of washing the nucleic acid array to remove residual sample (e.g., tissue or cells);
(v) after step (5) and before step (6) (e.g., after step (5) and before step pre-(6)), the method further comprises a step of amplifying (e.g., in-situ amplifying) the nucleic acid molecule comprising the spatial information tag; preferably, the nucleic acid molecule comprising the spatial information tag is attached to the solid support of the nucleic acid array.

32. The method according to any one of claims 26 to 31, wherein the sample is a tissue sample (e.g., a tissue section) or a single-cell sample (e.g., a single-cell suspension);
preferably, the tissue sample (e.g., tissue section) is prepared from fixed tissue, such as a formalin-fixed paraffin-embedded (FFPE) tissue, a deep-frozen tissue, or a fresh tissue.

33. The method according to any one of claims 26 to 32, wherein the nucleic acid derived from the sample to be tested is selected from the group consisting of: an RNA molecule (e.g., an mRNA), a target nucleic acid molecule (e.g., target DNA and/or RNA), a genomic nucleic acid fragment located in open region of chromatin, and any combination thereof.

34. The method according to any one of claims 26 to 33, wherein, in step (6), the analysis comprises sequencing and/or sequence-specific PCR;
preferably, prior to sequencing, the method further comprises a step of constructing a sequencing library from the nucleic acid molecule comprising the spatial information tag obtained in step (5) or an amplified product thereof.

35. The method according to any one of claims 26 to 34, wherein the method is used to detect the spatial information of an RNA (e.g., an mRNA) in cells of a sample.

36. The method according to claim 35, wherein, in step (2), the nucleic acid derived from the sample to be tested is an RNA (e.g., an mRNA) in the cells of the sample to be tested, and the capture sequence comprises a poly(dT) sequence or a random oligonucleotide sequence;
preferably, the step (3) comprises:
(a) under a condition allowing nucleic acid polymerization, using a nucleic acid molecule annealed to the capture sequence as a template, performing a nucleic acid polymerization reaction to extend the capture sequence, so as to generate a cDNA strand, wherein the cDNA strand comprises a cDNA sequence that is generated by a reverse transcription primed by the capture sequence and is complementary to the RNA (e.g., mRNA), and a 3' end overhang;
(b) under a condition allowing nucleic acid polymerization, annealing a template-switching sequence to the cDNA strand generated in (a), and continuing the nucleic acid polymerization reaction using the template-switching sequence as a template to generate the first extension product;
wherein the template-switching sequence comprises a common sequence, a complementary sequence to the 3' end overhang, and optionally an MID sequence; preferably, the MID sequence comprised in each template-switching sequence is different from one another;
the first extension product comprises: the capture sequence, the cDNA strand sequence or portion sequence thereof, and a complementary sequence to the common sequence;
preferably, the method further comprises, prior to step (4), a step of removing the template-switching sequence bound to the first extension product (e.g., removing the template-switching sequence bound to the first extension product by enzyme digestion or melting);
preferably, in step (4) of the method, the first universal sequence of the positioning probe is capable of annealing to a complementary sequence to the common sequence;
preferably, the positioning probe does not comprise an MID sequence, and the template-switching sequence comprises an MID sequence; or, the positioning probe comprises an MID sequence, and the template-switching sequence does not comprise an MID sequence, or, both the positioning probe and the template-switching sequence comprise MID sequences.

37. The method according to any one of claims 26 to 34, wherein the method is used for detecting spatial information of a target nucleic acid (e.g., a target DNA and/or RNA) of cells in a sample, and the target nucleic acid comprises a target nucleotide sequence;
preferably, in step (2), the nucleic acid derived from the sample to be tested comprises a target nucleotide sequence; preferably, the nucleic acid derived from the sample to be tested comprises the target nucleic acid and/or a nucleic acid molecule derived from the target nucleic acid, and the nucleic acid molecule derived from the target nucleic acid comprises the target nucleotide sequence;
preferably, in step (2), the capture sequence comprises a sequence capable of specifically recognizing the target nucleotide sequence;
preferably, in step (2), the capture sequence comprises a sequence capable of specifically recognizing a first segment of the target nucleotide sequence; in step (4), the first universal sequence of the positioning probe comprises a sequence capable of specifically recognizing a complementary sequence to a second segment of the target nucleotide sequence, or the first universal sequence comprises a random oligonucleotide sequence; preferably, in the target nucleotide sequence, the first segment is located 3' of the second segment;
preferably, in step (2), the first segment is present in a single-stranded region of a nucleic acid molecule that is derived from the sample to be tested and annealed to the capture sequence;
preferably, in step (2), the nucleic acid that is derived from the sample to be tested and annealed to the capture sequence is a single-stranded or double-stranded nucleic acid comprising a single-stranded region that comprises the first segment.

38. The method according to any one of claims 26 to 34, wherein the method is used for detecting spatial information of an RNA (e.g., an mRNA) and a target nucleic acid (e.g., a target DNA and/or RNA) of cells in a sample, wherein the target nucleic acid comprises a target nucleotide sequence;
preferably, the nucleic acid array comprises a first capture probe capable of capturing an RNA (e.g., an mRNA), and a second capture probe capable of capturing a nucleic acid molecule comprising a target nucleotide sequence; the first capture probe comprises a first capture sequence, the first capture sequence comprising a poly(dT) sequence or a random oligonucleotide sequence; the second capture probe comprises a second capture sequence, the second capture sequence comprising a sequence capable of specifically recognizing the target nucleotide sequence;
preferably, in step (2), the nucleic acid derived from the sample to be tested comprises an RNA (e.g., an mRNA) derived from the sample to be tested and a nucleic acid molecule comprising a target nucleotide sequence; preferably, the nucleic acid molecule comprising the target nucleotide sequence comprises the target nucleic acid and/or a nucleic acid molecule derived from the target nucleic acid;
preferably, the step (3) comprises:
(A)
(a) under a condition allowing nucleic acid polymerization, using a nucleic acid molecule annealed to the first capture sequence as a template, performing a nucleic acid polymerization reaction to extend the first capture sequence, so as to generate a cDNA strand, wherein the cDNA strand comprises a cDNA sequence that is generated by a reverse transcription primed by the first capture sequence and is complementary to the RNA (e.g., mRNA), and a 3' end overhang;
(b) under a condition allowing nucleic acid polymerization, annealing a template-switching sequence to the cDNA strand generated in (a), and continuing the nucleic acid polymerization reaction using the template-switching sequence as a template to generate a first extension product I;
wherein, the template-switching sequence comprises a common sequence, a complementary sequence to the 3' end overhang, and optionally an MID sequence; preferably, the MID sequence comprised in each template-switching sequence is different from one another;
and,
(B) under a condition allowing nucleic acid polymerization, using a nucleic acid molecule annealed to the second capture sequence as a template, performing a nucleic acid polymerization reaction to extend the second capture sequence, so as to generate a first extension product II, wherein the first extension product II comprises the second capture sequence and a complementary sequence to the nucleic acid molecule or portion sequence thereof annealed to the second capture sequence;
wherein, the (B) and the (A) are performed in any order; for example, the (B) is performed before or after the (A), or the (B) is performed simultaneously with the (A) (e.g., in the same reaction system);
preferably, in step (4), the positioning probe comprises a first universal sequence I capable of annealing to the first extension product I, and a first universal sequence II capable of annealing to the first extension product II; preferably, the first universal sequence I and the first universal sequence II coexist on the same positioning probe, or are present on different positioning probes, respectively;
preferably, the nucleic acid array comprises a first positioning probe comprising the first universal sequence I and a second positioning probe comprising the first universal sequence II;
preferably, in step (4) of the method, the first universal sequence I is capable of annealing to a complementary sequence to the common sequence;
preferably, in step (2) of the method, the second capture sequence comprises a sequence capable of specifically recognizing a first segment of the target nucleotide sequence; in step (4), the first universal sequence II comprises a sequence capable of specifically recognizing a complementary sequence to a second segment of the target nucleotide sequence, or the first universal sequence II comprises a random oligonucleotide sequence; preferably, in the target nucleotide sequence, the first segment is located 3' of the second segment;
preferably, the method further comprises, prior to step (4), a step of removing the template-switching sequence bound to the first extension product I (e.g., removing the template-switching sequence bound to the first extension product I by enzymatic digestion or melting);
preferably, in step (2), the first segment is present in a single-stranded region of a nucleic acid molecule that is derived from the sample to be tested and annealed to the second capture sequence;
preferably, in step (2), the nucleic acid that is derived from the sample to be tested and annealed with the second capture sequence is a single-stranded or double-stranded nucleic acid comprising a single-stranded region that comprises the first segment;
preferably, the first positioning probe does not comprise an MID sequence, and the template-switching sequence comprises an MID sequence; or, the first positioning probe comprises an MID sequence, and the template-switching sequence does not comprise an MID sequence; or, both the first positioning probe and the template-switching sequence comprise MID sequences.

39. A kit, which comprises the nucleic acid array according to any one of claims 1 to 8.

40. The kit according to claim 39, wherein a capture sequence of the nucleic acid array comprises a poly(dT) sequence or a random oligonucleotide sequence;
preferably, the kit further comprises a template-switching sequence, wherein the template-switching sequence comprises a common sequence, a complementary sequence to a 3' end overhang of cDNA, and optionally an MID sequence; preferably, the MID sequence comprised in each template-switching sequence is different from one another; preferably, the 3' end overhang of cDNA has a length of at least 1, at least 2, at least 3, at least 4, at least 5, at least 6, at least 7, at least 8, at least 9, at least 10, 1 to 10, 1 to 5, or 2 to 10 nucleotides; preferably, the 3' end overhang of cDNA is an overhang consisting of 2 to 5 cytosine nucleotides (e.g., a CCC overhang);
preferably, a complementary sequence to the common sequence of the template-switching sequences is capable of annealing to the first universal sequence of the positioning probe of the nucleic acid array;
preferably, the positioning probe does not comprise an MID sequence, and the template-switching sequence comprises an MID sequence; or, the positioning probe comprises an MID sequence, and the template-switching sequence does not comprise an MID sequence; or, both the positioning probe and the template-switching sequence comprises MID sequences.

41. The kit according to claim 39, wherein the capture sequence of the nucleic acid array comprises a sequence capable of specifically recognizing a target nucleotide sequence comprised in a target nucleic acid (e.g., a certain target DNA and/or RNA);
preferably, the first universal sequence comprises a sequence capable of specifically recognizing a complementary sequence to the target nucleotide sequence;
preferably, the capture sequence comprises a sequence capable of specifically recognizing a first segment of the target nucleotide sequence, the first universal sequence of the positioning probe of the nucleic acid array comprises a sequence capable of specifically recognizing a complementary sequence to a second segment of the target nucleotide sequence, or, the first universal sequence comprises a random oligonucleotide sequence;
preferably, in the target nucleotide sequence, the first segment is located 3' of the second segment.

42. The kit according to claim 39, wherein the nucleic acid array comprises a first capture probe and a second capture probe; the first capture probe comprises a first capture sequence, wherein the first capture sequence comprises a poly(dT) sequence or a random oligonucleotide sequence; the second capture probe comprises a second capture sequence, wherein the second capture sequence comprises a sequence capable of specifically recognizing a target nucleotide sequence comprised in a target nucleic acid (e.g., a certain target DNA and/or RNA);
preferably, the kit further comprises a template-switching sequence, wherein the template-switching sequence comprises a common sequence, a complementary sequence to a 3' end overhang of cDNA, and optionally an MID sequence; preferably, the MID sequence comprised in each template-switching sequence is different from one another; preferably, the 3' end overhang of cDNA has a length of at least 1, at least 2, at least 3, at least 4, at least 5, at least 6, at least 7, at least 8, at least 9, at least 10, 1 to 10, 1 to 5, or 2 to 10 nucleotides; preferably, the 3' end overhang of cDNA is an overhang consisting of 2 to 5 cytosine nucleotides (e.g., a CCC overhang);
preferably, the positioning probe of the nucleic acid array comprises a first universal sequence I and a first universal sequence II; wherein the first universal sequence I is capable of annealing to a complementary sequence to the common sequence, and the first universal sequence II comprises a sequence capable of specifically recognizing a complementary sequence to the target nucleotide sequence, or the first universal sequence II comprises a random oligonucleotide sequence; preferably, the first universal sequence I and the first universal sequence II coexist on the same positioning probe, or they are present on different positioning probes;
preferably, the nucleic acid array comprises a first positioning probe that comprises the first universal sequence I, and a second positioning probe that comprises the first universal sequence II; preferably, the first targeting probe does not comprise an MID sequence, and the template-switching sequence comprises an MID sequence; or, the first targeting probe comprises an MID sequence, and the template-switching sequence does not comprise an MID sequence; or, both the first targeting probe and the template-switching sequence comprise MID sequences;
preferably, the second capture sequence comprises a sequence capable of specifically recognizing a first segment of the target nucleotide sequence, and the first universal sequence II comprises a sequence capable of specifically recognizing a complementary sequence to a second segment of the target nucleotide sequence; or, the first universal sequence II comprises a random oligonucleotide sequence; preferably, in the target nucleotide sequence, the first segment is located 3' of the second segment.

43. The kit according to any one of claims 39 to 42, wherein the first universal sequence comprised in the positioning probe of the nucleic acid array is or is not located at the 3' end of the positioning probe, and/or, the 3' end of the first universal sequence of the positioning probe of the nucleic acid array is or is not blocked;
preferably, the kit further comprises: a reagent for nucleic acid hybridization, a reagent for nucleic acid extension, a reagent for nucleic acid amplification, a reagent for recovering or purifying nucleic acid, a reagent for constructing a transcriptome sequencing library, a reagent for sequencing (e.g., second-generation sequencing or third-generation sequencing), or any combination thereof.

44. Use of the nucleic acid array according to any one of claims 1 to 8 or the kit according to any one of claims 39 to 43 for constructing a nucleic acid molecular library, for performing nucleic acid sequencing, or for detecting spatial information of a nucleic acid in a sample.
